# EUROPEAN PATENT SPECIFICATION

(11) **EP 2 067 788 B1**
(45) Date of publication and mention of the grant of the patent: **22.07.2015**
(21) Application number: 09151233.5
(22) Date of filing: 18.05.1999
(51) Int. Cl.: C07K 16/30, C07K 16/00, C07K 16/18, C12N 15/63, C12N 15/10

(54) **Fab fragment libraries and methods for their use**
FAB-Fragmentbibliotheken und Verwendungsverfahren dafür
Bibliothèque des fragments Fab et méthodes d'utilisation

(43) Date of publication of application: 10.06.2009
(62) Divisional of application: 99201558.6
(73) Proprietor: Dyax Corp., Burlington, MA 01803 (US)
(72) Inventor: Hoogenboom, Hendricus Renerus Jacobus Mattheus, 6214 AE Maastricht (NL)
(74) Representative: Wichmann, Hendrik

(56) References cited:
- EP-A1- 0 844 306
- EP-A1- 0 866 136
- WO-A1-90/14443
- WO-A1-92/01047
- WO-A1-94/05781
- US-A- 5 427 908
- EVA ANDERSSON ET AL: "A tandem repeat of MUC1 core protein induces a weak in vitro inmune response in human B cells" CANCER IMMUNOLOGY, IMMUNOTHERAPY, vol. 47, no. 5, January 1999 (1999-01), pages 249-256, XP002571641
- DE HAARD H J ET AL: "A large non-immunized human Fab fragment phage library that permits rapid isolation and kinetic analysis of high affinity antibodies." JOURNAL OF BIOLOGICAL CHEMISTRY, vol. 274, no. 26, 25 June 1999 (1999-06-25), pages 18218-18230, XP002128301
- HUSE W D ET AL: "GENERATION OF A LARGE COMBINATORIAL LIBRARY OF THE IMMUNOGLOBULIN REPERTOIRE IN PHAGE LAMBDA" SCIENCE, AMERICAN ASSOCIATION FOR THE ADVANCEMENT OF SCIENCE, WASHINGTON, DC; US, vol. 246, 8 December 1989 (1989-12-08), pages 1275-1281, XP002949267 ISSN: 0036-8075
- BARBAS C F III ET AL: "ASSEMBLY OF COMBINATORIAL ANTIBODY LIBRARIES ON PHAGE SURFACES THE GENE III SITE" PROCEEDINGS OF THE NATIONAL ACADEMY OF SCIENCES OF THE UNITED STATES OF AMERICA, vol. 88, no. 18, 1991, pages 7978-7982, XP002571633 ISSN: 0027-8424
- SIEGEL DON L ET AL: "Expression and characterization of recombinant anti-Rh(D) antibodies on filamentous phage: A model system for isolating human red blood cell antibodies by repertoire cloning" BLOOD, vol. 83, no. 8, 1994, pages 2334-2344, XP002571634 ISSN: 0006-4971
- DOLEZAL O ET AL: "Escherichia coli expression of a bifunctional Fab-peptide epitope reagent for the rapid diagnosis of HIV-1 and HIV-2" IMMUNOTECHNOLOGY, ELSEVIER SCIENCE PUBLISHERS BV, NL, vol. 1, no. 3, 1 December 1995 (1995-12-01), pages 197-209, XP004052722 ISSN: 1380-2933
- RADER CHRISTOPH ET AL: "Phage display of combinatorial antibody libraries" CURRENT OPINION IN BIOTECHNOLOGY, vol. 8, no. 4, 1997, pages 503-508, XP002571635 ISSN: 0958-1669
- BOEKE J D ET AL: "A PROKARYOTIC MEMBRANE ANCHOR SEQUENCE CARBOXYL TERMINUS OF BACTERIO PHAGE F-1 GENE III PROTEIN RETAINS IT IN THE MEMBRANE", PROCEEDINGS OF THE NATIONAL ACADEMY OF SCIENCES OF THE UNITED STATES OF AMERICA, vol. 79, no. 17, 1982, pages 5200-5204, ISSN: 0027-8424

### SEQUENCE LISTING

<110> Dyax Corp.
<120> Novel Fab fragment libraries and methods for their use
<130> P24015EP01
<140>
   <141> 1999-05-18
<160> 69
<170> PatentIn Ver. 2.1
<210> 1
   <211> 24
   <212> DNA
   <213> Artificial Sequence
<220>
   <223> Description of Artificial Sequence: oligonucleotide
<400> 1
   tggaagaggc acgttctttt cttt 24
<210> 2
   <211> 24
   <212> DNA
   <213> Artificial Sequence
<220>
   <223> Description of Artificial Sequence: oligonucleotide
<400> 2
   acactctccc ctgttgaagc tctt 24
<210> 3
   <211> 23
   <212> DNA
   <213> Artificial sequence
<220>
   <223> Description of Artificial Sequence: oligonucleotide
<400> 3
   tgaacattct gtaggggcca ctg 23
<210> 4
   <211> 23
   <212> DNA
   <213> Artificial Sequence
<220>
   <223> Description of Artificial Sequence: oligonucleotide
<400> 4
   agagcattct gcaggggcca ctg 23
<210> 5
   <211> 23
   <212> DNA
   <213> Artificial Sequence
<220>
   <223> Description of Artificial sequence: oligonucleotide
<400> 5
   cagrtgcagc tggtgcartc tgg 23
<210> 6
   <211> 23
   <212> DNA
   <213> Artificial Sequence
<220>
   <223> Description of Artificial sequence: oligonucleotide
<400> 6
   saggtccagc tggtrcagtc tgg 23
<210> 7
   <211> 23
   <212> DNA
   <213> Artificial Sequence
<220>
   <223> Description of Artificial Sequence: oligonucleotide
<400> 7
   cagrtcacct tgaaggagtc tgg 23
<210> 8
   <211> 23
   <212> DNA
   <213> Artificial Sequence
<220>
   <223> Description of Artificial Sequence: oligonucleotide
<400> 8
   saggtgcagc tggtggagtc tgg 23
<210> 9
   <211> 23
   <212> DNA
   <213> Artificial Sequence
<220>
   <223> Description of Artificial Sequence: oligonucleotide
<400> 9
   gaggtgcagc tggtggagwc ygg 23
<210> 10
   <211> 23
   <212> DNA
   <213> Artificial sequence
<220>
   <223> Description of Artificial Sequence: oligonucleotide
<400> 10
   caggtgcagc tacagcagtg ggg 23
<210> 11
   <211> 23
   <212> DNA
   <213> Artificial Sequence
<220>
   <223> Description of Artificial Sequence: oligonucleotide
<400> 11
   cagstgcagc tgcaggagtc sgg 23
<210> 12
   <211> 23
   <212> DNA
   <213> Artificial sequence
<220>
   <223> Description of Artificial Sequence: oligonucleotide
<400> 12
   gargtgcagc tggtgcagtc tgg 23
<210> 13
   <211> 23
   <212> DNA
   <213> Artificial sequence
<220>
   <223> Description of Artificial sequence: oligonucleotide
<400> 13
   caggtacagc tgcagcagtc agg 23
<210> 14
   <211> 23
   <212> DNA
   <213> Artificial Sequence
<220>
   <223> Description of Artificial Sequence: oligonucleotide
<400> 14
   gacatccagw tgacccagtc tcc 23
<210> 15
   <211> 23
   <212> DNA
   <213> Artificial Sequence
<220>
   <223> Description of Artificial Sequence: oligonucleotide
<400> 15
   gatgttgtga tgactcagtc tcc 23
<210> 16
   <211> 23
   <212> DNA
   <213> Artificial sequence
<220>
   <223> Description of Artificial sequence: oligonucleotide
<400> 16
   gaaattgtgw tgacrcagtc tcc 23
<210> 17
   <211> 23
   <212> DNA
   <213> Artificial sequence
<220>
   <223> Description of Artificial Sequence: oligonucleotide
<400> 17
   gatattgtga tgacccacac tcc 23
<210> 18
   <211> 23
   <212> DNA
   <213> Artificial Sequence
<220>
   <223> Description of Artificial sequence: oligonucleotide
<400> 18
   gaaacgacac tcacgcagtc tcc 23
<210> 19
   <211> 23
   <212> DNA
   <213> Artificial Sequence
<220>
   <223> Description of Artificial sequence: oligonucleotide
<400> 19
   gaaattgtgc tgactcagtc tcc 23
<210> 20
   <211> 23
   <212> DNA
   <213> Artificial sequence
<220>
   <223> Description of Artificial sequence: oligonucleotide
<400> 20
   cagtctgtgc tgactcagcc acc 23
<210> 21
   <211> 23
   <212> DNA
   <213> Artificial Sequence
<220>
   <223> Description of Artificial sequence: oligonucleotide
<400> 21
<210> 22
   <211> 23
   <212> DNA
   <213> Artificial sequence
<220>
   <223> Description of Artificial sequence: oligonucleotide
<400> 22
   cagtctgtcg tgacgcagcc gcc 23
<210> 23
   <211> 21
   <212> DNA
   <213> Artificial Sequence
<220>
   <223> Description of Artificial sequence: oligonucleotide
<400> 23
   cartctgccc tgactcagcc t 21
<210> 24
   <211> 23
   <212> DNA
   <213> Artificial Sequence
<220>
   <223> Description of Artificial Sequence: oligonucleotide
<400> 24
   tcctatgwgc tgactcagcc acc 23
<210> 25
   <211> 23
   <212> DNA
   <213> Artificial Sequence
<220>
   <223> Description of Artificial sequence: oligonucleotide
<400> 25
   tcttctgagc tgactcagga ccc 23
<210> 26
   <211> 23
   <212> DNA
   <213> Artificial sequence
<220>
   <223> Description of Artificial Sequence: oligonucleotide
<400> 26
   cacgttatac tgactcaacc gcc 23
<210> 27
   <211> 23
   <212> DNA
   <213> Artificial sequence
<220>
   <223> Description of Artificial Sequence: oligonucleotide
<400> 27
   caggctgtgc tgactcagcc gtc 23
<210> 28
   <211> 23
   <212> DNA
   <213> Artificial Sequence
<220>
   <223> Description of Artificial Sequence: oligonucleotide
<400> 28
   aattttatgc tgactcagcc cca 23
<210> 29
   <211> 23
   <212> DNA
   <213> Artificial sequence
<220>
   <223> Description of Artificial Sequence: oligonucleotide
<400> 29
   cagrctgtgg tgacycagga gcc 23
<210> 30
   <211> 23
   <212> DNA
   <213> Artificial sequence
<220>
   <223> Description of Artificial Sequence: oligonucleotide
<400> 30
   cwgcctgtgc tgactcagcc mcc 23
<210> 31
   <211> 51
   <212> DNA
   <213> Artificial Sequence
<220>
   <223> Description of Artificial Sequence: oligonucleotide
<400> 31
   accgcctcca ccgggcgcgc cttattaaca ctctcccctg ttgaagctct t 51
<210> 32
   <211> 50
   <212> DNA
   <213> Artificial Sequence
<220>
   <223> Description of Artificial sequence: oligonucleotide
<400> 32
   accgcctcca ccgggcgcgc cttattatga acattctgta ggggccactg 50
<210> 33
   <211> 50
   <212> DNA
   <213> Artificial Sequence
<220>
   <223> Description of Artificial sequence: oligonucleotide
<400> 33
   accgcctcca ccgggcgcgc cttattaaga gcattctgca ggggccactg 50
<210> 34
   <211> 56
   <212> DNA
   <213> Artificial Sequence
<220>
   <223> Description of Artificial sequence: oligonucleotide
<400> 34
   gtcctcgcaa ctgcggccca gccggccatg gcccagrtgc agctggtgca rtctgg 56
<210> 35
   <211> 56
   <212> DNA
   <213> Artificial sequence
<220>
   <223> Description of Artificial sequence: oligonucleotide
<400> 35
   gtcctcgcaa ctgcggccca gccggccatg gccsaggtcc agctggtrca gtctgg 56
<210> 36
   <211> 56
   <212> DNA
   <213> Artificial Sequence
<220>
   <223> Description of Artificial Sequence: oligonucleotide
<400> 36
   gtcctcgcaa ctgcggccca gccggccatg gcccagrtca ccttgaagga gtctgg 56
<210> 37
   <211> 56
   <212> DNA
   <213> Artificial sequence
<220>
   <223> Description of Artificial sequence: oligonucleotide
<400> 37
   gtcctcgcaa ctgcggccca gccggccatg gccsaggtgc agctggtgga gtctgg 56
<210> 38
   <211> 56
   <212> DNA
   <213> Artificial sequence
<220>
   <223> Description of Artificial Sequence: oligonucleotide
<400> 38
   gtcctcgcaa ctgcggccca gccggccatg gccgaggtgc agctggtgga gwcygg 56
<210> 39
   <211> 56
   <212> DNA
   <213> Artificial Sequence
<220>
   <223> Description of Artificial sequence: oligonucleotide
<400> 39
   gtcctcgcaa ctgcggccca gccggccatg gcccaggtgc agctacagca gtgggg 56
<210> 40
   <211> 56
   <212> DNA
   <213> Artificial Sequence
<220>
   <223> Description of Artificial sequence: oligonucleotide
<400> 40
   gtcctcgcaa ctgcggccca gccggccatg gcccagstgc agctgcagga gtcsgg 56
<210> 41
   <211> 56
   <212> DNA
   <213> Artificial Sequence
<220>
   <223> Description of Artificial Sequence: oligonucleotide
<400> 41
   gtcctcgcaa ctgcggccca gccggccatg gccgargtgc agctggtgca gtctgg 56
<210> 42
   <211> 56
   <212> DNA
   <213> Artificial sequence
<220>
   <223> Description of Artificial Sequence: oligonucleotide
<400> 42
   gtcctcgcaa ctgcggccca gccggccatg gcccaggtac agctgcagca gtcagg 56
<210> 43
   <211> 24
   <212> DNA
   <213> Artificial Sequence
<220>
   <223> Description of Artificial Sequence: oligonucleotide
<400> 43
   tgaggagacg gtgaccaggg tgcc 24
<210> 44
   <211> 24
   <212> DNA
   <213> Artificial Sequence
<220>
   <223> Description of Artificial sequence: oligonucleotide
<400> 44
   tgaagagacg gtgaccattg tccc 24
<210> 45
   <211> 24
   <212> DNA
   <213> Artificial Sequence
<220>
   <223> Description of Artificial sequence: oligonucleotide
<400> 45
   tgaggagacg gtgaccaggg ttcc 24
<210> 46
   <211> 24
   <212> DNA
   <213> Artificial sequence
<220>
   <223> Description of Artificial sequence: oligonucleotide
<400> 46
   tgaggagacg gtgaccgtgg tccc 24
<210> 47
   <211> 44
   <212> DNA
   <213> Artificial Sequence
<220>
   <223> Description of Artificial sequence: oligonucleotide
<400> 47
   accgcctcca ccagtgcact tgacatccag wtgacccagt ctcc 44
<210> 48
   <211> 44
   <212> DNA
   <213> Artificial sequence
<220>
   <223> Description of Artificial sequence: oligonucleotide
<400> 48
   accgcctcca ccagtgcact tgatgttgtg atgactcagt ctcc 44
<210> 49
   <211> 44
   <212> DNA
   <213> Artificial Sequence
<220>
   <223> Description of Artificial sequence: oligonucleotide
<400> 49
   accgcctcca ccagtgcact tgaaattgtg wtgacrcagt ctcc 44
<210> 50
   <211> 44
   <212> DNA
   <213> Artificial sequence
<220>
   <223> Description of Artificial Sequence: oligonucleotide
<400> 50
   accgcctcca ccagtgcact tgatattgtg atgacccaca ctcc 44
<210> 51
   <211> 44
   <212> DNA
   <213> Artificial Sequence
<220>
   <223> Description of Artificial sequence: oligonucleotide
<400> 51
   accgcctcca ccagtgcact tgaaacgaca ctcacgcagt ctcc 44
<210> 52
   <211> 44
   <212> DNA
   <213> Artificial Sequence
<220>
   <223> Description of Artificial Sequence: oligonucleotide
<400> 52
   accgcctcca ccagtgcact tgaaattgtg ctgactcagt ctcc 44
<210> 53
   <211> 41
   <212> DNA
   <213> Artificial Sequence
<220>
   <223> Description of Artificial Sequence: oligonucleotide
<400> 53
   accgcctcca ccagtgcaca gtctgtgctg actcagccac c 41
<210> 54
   <211> 41
   <212> DNA
   <213> Artificial Sequence
<220>
   <223> Description of Artificial Sequence: oligonucleotide
<400> 54
   accgcctcca ccagtgcaca gtctgtgytg acgcagccgc c 41
<210> 55
   <211> 41
   <212> DNA
   <213> Artificial Sequence
<220>
   <223> Description of Artificial Sequence: oligonucleotide
<400> 55
   accgcctcca ccagtgcaca gtctgtcgtg acgcagccgc c 41
<210> 56
   <211> 39
   <212> DNA
   <213> Artificial Sequence
<220>
   <223> Description of Artificial sequence: oligonucleotide
<400> 56
   accgcctcca ccagtgcaca rtctgccctg actcagcct 39
<210> 57
   <211> 44
   <212> DNA
   <213> Artificial Sequence
<220>
   <223> Description of Artificial sequence: oligonucleotide
<400> 57
   accgcctcca ccagtgcact ttcctatgwg ctgactcagc cacc 44
<210> 58
   <211> 44
   <212> DNA
   <213> Artificial Sequence
<220>
   <223> Description of Artificial sequence: oligonucleotide
<400> 58
   accgcctcca ccagtgcact ttcttctgag ctgactcagg accc 44
<210> 59
   <211> 41
   <212> DNA
   <213> Artificial sequence
<220>
   <223> Description of Artificial sequence: oligonucleotide
<400> 59
   accgcctcca ccagtgcaca cgttatactg actcaaccgc c 41
<210> 60
   <211> 41
   <212> DNA
   <213> Artificial Sequence
<220>
   <223> Description of Artificial sequence: oligonucleotide
<400> 60
   accgcctcca ccagtgcaca ggctgtgctg actcagccgt c 41
<210> 61
   <211> 44
   <212> DNA
   <213> Artificial sequence
<220>
   <223> Description of Artificial sequence: oligonucleotide
<400> 61
   accgcctcca ccagtgcact taattttatg ctgactcagc ccca 44
<210> 62
   <211> 41
   <212> DNA
   <213> Artificial Sequence
<220>
   <223> Description of Artificial Sequence: oligonucleotide
<400> 62
   accgcctcca ccagtgcaca grctgtggtg acycaggagc c 41
<210> 63
   <211> 41
   <212> DNA
   <213> Artificial sequence
<220>
   <223> Description of Artificial Sequence: oligonucleotide
<400> 63
   accgcctcca ccagtgcacw gcctgtgctg actcagccmc c 41
<210> 64
   <211> 89
   <212> DNA
   <213> Artificial Sequence
<220>
   <223> Description of Artificial Sequence: Fragment of pCES1 comprising signal sequence, restriction sites, 5À□□À part of human Ck gene
<400> 64
<210> 65
   <211> 163
   <212> DNA
   <213> Artificial Sequence
<220>
   <223> Description of Artificial sequence: Fragment of pCES1
<400> 65
   ggagagtgtt aataaggcgc gccaattcta tttcaaggag acagtcataa tgaaatacct 60
   attgcctacg gcagccgctg gattgttatt actcgcggcc cagccggcca tggcccaggt 120
   gcagctgcag gagagcgggg tcaccgtctc aagcgcctcc acc                  163
<210> 66
   <211> 96
   <212> DNA
   <213> Artificial Sequence
<220>
   <223> Description of Artificial sequence: Fragment of pCES1 comprising 3À□□À part of human CH1 gene, restriction site, hexahistidine tag, cMyc tag, stop codon and 5À□□À part of of gene III
<400> 66
   aaatcttgtg cggccgcaca tcatcatcat catcacgggg ccgcagaaca aaaactcatc 60
   tcagaagagg atctgaatgg ggccgcatag actgtt                           96
<210> 67
   <211> 30
   <212> PRT
   <213> Artificial Sequence
<220>
   <223> Description of Artificial sequence: Fragment of pCES1
<400> 67
   Leu Phe Ala Ile Pro Leu Val Val Pro Phe Tyr Ser His Ser Ala Gln
   1               5                   10                  15
   Val Gln Leu Gln Val Asp Leu Glu Ile Lys Arg Gly Thr Val
               20                  25                  30
<210> 68
   <211> 38
   <212> PRT
   <213> Artificial Sequence
<220>
   <223> Description of Artificial sequence: Fragment encoded by pCES1 comprising signal sequence and amino terminal part of human CH1 gene product
<400> 68
   Met Lys Tyr Leu Leu Pro Thr Ala Ala Ala Gly Leu Leu Leu Leu Ala
   1               5                   10                  15
   Ala Gln Pro Ala Met Ala Gln Val Gln Leu Gln Glu Ser Gly Val Thr
               20                  25                  30
   Val Ser Ser Ala Ser Thr
           35
<210> 69
   <211> 29
   <212> PRT
   <213> Artificial Sequence
<220>
   <223> Description of Artificial sequence: Fragment encoded by pCES1 comprising carboxy terminal part of human CH1 gene product, hexahistidine tag and cMyc tag
<400> 69
   Lys Ser Cys Ala Ala Ala His His His His His His Gly Ala Ala Glu
   1               5                   10                  15
   Gln Lys Leu Ile Ser Glu Glu Asp Leu Asn Gly Ala Ala
               20                  25

## Description

This invention relates in general to methods for constructing phage display libraries of human Fab fragments, as defined in the claims.

Display on filamentous phage in combination with selection forms a powerful tool for the identification of peptide- or protein-based drugs (Winter et al., 1994; Clackson et al., 1994). Of these, antibodies are especially of interest, due to their capacity to recognize a variety of targets with high specificity and affinity. Particularly the use of partial or complete human antibodies, which elicit no or minimal immune response when administered to patients, is yielding an increasing list of FDA-approved protein-based drugs (Holliger et al. 1998). Phage display technology enables the generation of large repertoires of human antibodies (Marks et al., 1991, Hoogenboom et al., 1992; Griffiths et al., 1993; Vaughan et al., 1996), and biopanning procedures permit the selection of individual antibodies with a desired specificity.

Key to the success of the technology were two critical observations: (i) the expression of functional antibody fragments by secretion into the periplasm of *E. coli* (Better et al., 1988; Skerra et al., 1988), and, (ii) the rapid access to variable region gene pools by the polymerase chain reaction (Larrick et al., 1989; Ward et al., 1989; Marks et al., 1991). For the construction of antibody libraries, V-genes are amplified from B-cell cDNA and heavy and light chain genes are randomly combined and cloned to encode a combinatorial library of single-chain Fv (scFv) or Fab antibody fragments (Marks et al., 1991; Clackson et al. 1991; Persson et al., 1991; Orum et al., 1993). The natural primary (unselected) antibody repertoire within B-cells contains a large array of antibodies that recognize a variety of antigens; this array can be cloned as a 'naïve' repertoire of rearranged genes, by harvesting the V-genes from the IgM mRNA of B-cells of unimmunized human donors, isolated from peripheral blood lymphocytes (Marks et al., 1991), bone marrow or tonsils (Vaughan et al., 1996), or from similar animal sources (Gram et al., 1992). This procedure provides access to antibodies that have not yet encountered antigen, although the frequency of those genuine 'germline' antibodies will depend heavily on the source of B-cells (Klein et al., 1997). A single 'naïve' library, if sufficiently large and diverse, can indeed be used to generate antibodies to a large panel of antigens, including self, non-immunogenic and relatively toxic antigens (Griffiths et al., 1993; Marks et al., 1991). In a different approach, antibodies may be built artificially, by *in vitro* assembly of V-gene segments and D/J segments, yielding 'synthetic' antibodies (Hoogenboom et al., 1992). A major drawback of these procedures is that from the initial'naïve' and 'synthetic' libraries, only moderate affinity antibodies were isolated (Marks et al., 1991; Nissim et al., 1994). Over the last few years more efficient techniques have been developed to build larger libraries of antibody fragments, using sophisticated *in vivo* recombination methods (Griffiths et al., 1993) or brute force cloning procedures (Vaughan et al., 1996; Sheets et al., 1998). Such large libraries have yielded a greater number of human antibodies per antigen tested, with an average much higher affinity (up to sub-nanomolar). However, technical restrictions on the size of libraries that may be obtained or handled in selection, the loss of library diversity upon library amplification, and the relatively long downstream analysis path of the selected antibodies, i.e., large scale affinity analysis, have limited the spread of these libraries as generic tools in antibody generation.

Most large libraries made to date use the single chain format for display on phage (Vaughan et al., 1996; Sheets et al. 1998). One report described the use of a human naive Fab library on phage (not permitting immediate screening of selected soluble Fab fragments) (Griffiths et al., 1994). scFv's have the tendency to form dimers and higher order multimers in a clone-dependent and relatively unpredictable way (Weidner, et al. 1992; Holliger, et al. 1993; Marks et al., 1993). As a consequence, the affinity assay used (such as BIAcore analysis) often necessitates purification of the selected antibody fragments. For example, ranking for off-rates using BIAcore is not easily possible with unpurified scFv fragments; the monomeric fraction of selected scFv clones first needs to be purified by affinity chromatography and gel-filtration (Sheets et al., 1998; Schier et al., 1996).

As was postulated and observed by Griffiths and colleagues (Griffiths et al., 1994), the size of the antibody library dictates the probability of the selection of high affinity antibodies to the antigen. Comparison of the first naïve scFv repertoire containing 2.9x10⁷ clones (Marks et al., 1991), with a recently constructed scFv repertoire of approximately 10¹⁰ clones (Vaughan et al., 1996; Sheets et al. 1998), confirms this postulation: increasing the library size 500-fold resulted in approximately 100-fold higher affinities. This increase is caused by lowering the off-rates from 10⁻¹-10⁻² s⁻¹ for fragments selected from the smaller sized library to 10⁻³-10⁻⁴ s⁻¹ for those from the larger library.

Huse et al. Science 246:1275-1281 (1989) discloses the generation of a large combinatorial library of the immunoglobulin repertoire in phage Lambda.

It is an object of the invention to create a Fab library that is a valuable source of antibodies for many different targets, and which will play a vital role in target discovery and validation in the area of functional genomics.

The invention provides a a method of making a Fab library, the library comprising a plurality of vectors wherein each vector of the plurality of vectors comprises:
- a first cloning region and second cloning region, wherein
- each cloning region comprises at least one, for the vector unique, restriction enzyme cleavage site,
- each cloning region being 5' flanked by a ribosome binding site and a signal sequence,
- a polynucleotide encoding an anchor region, located 3' of the second cloning region,
- a polynucleotide encoding a constant heavy chain region or portion thereof located between the vector unique restriction enzyme cleavage site of the second cloning region and the anchor region,
- a polynucleotide encoding a tag,
the method copmprising:
introducing into the first cloning region a member of a first plurality of variable polynucleotides, said plurality of variable polynucleotides encoding a first plurality of polypeptides, wherein the member of the first plurality of polynucleotides encodes a polypeptide comprising an antibody light chain variable region possibly followed by an antibody light chain constant region,
separately introducing into the second cloning region of each vector a member of a second plurality of variable polynucleotides, said plurality of variable polynucleotides encoding a second plurality of polypeptides, wherein the member of the second plurality of polynucleotides encodes a polypeptide comprising an antibody heavy chain variable region, and
introducing each of the vectors in a host cell to provide a plurality of capsid particles each comprising a member of the first plurality of variable polynucleotides and a member of the second plurality of polynucleotides, to thereby make the library of antibodies or fragments thereof.

It is to be understood that the term "for the vector unique restriction enzyme cleavage site" refers to the presence of one of such a restriction site in the vector sequence, without taking into account the possible presence of such a site on the above-mentioned first and/or second polynucleotides encoding a complete antibody variable region or part of an antibody variable region, possibly followed by a complete antibody constant region or part of an antibody constant region. The said first and second polynucleotides may comprise restriction sites identical to the "unique" site. This means that the said restriction site was "unique" before both first and second polynucleotide sequences were cloned into the vector.

The first and second variable polynucleotides are preferably cloned in the cloning region in a predetermined orientation. Therefore, in case the cloning region comprises a single unique restriction site, this site is preferably of such a type that non-identical restriction ends are generated, such as e.g, generated by the restriction enzyme S*fi*I. However, the cloning region may comprise two or more unique restriction sites, so that the variable polynucleotides can be conveniently cloned as a restriction fragment that has the corresponding ends.

Preferably, in the vector used in the method according to the invention, the first and second cloning regions, both ribosomal binding sites, signal sequences and the anchor sequence are part of a single polylinker sequence. Both cloning regions may therefore be part of a single cassette, comprising the first cloning region, 5' flanked by a ribosomal binding site and a signal sequence, lying adjacent to the second cloning region, also 5' flanked by its corresponding ribosomal binding site and a signal sequence, and 3' flanked by the anchor sequence.

Preferably, the first plurality of variable polynucleotides are V_{L} polynucleotides, and the second plurality of variable polynuclotides are V_{H} polynucleotides. More preferably, the V_{L} polynucleotides are V_{κ} polynucleotides, V_{κ}C_{κ} polynucleotides, V_{λ} polynucleotides, V_{λ}C_{λ} polynucleotides, a mixture of V_{κ} and V_{λ} polynucleotides, or a mixture of V_{κ}C_{κ} and V_{λ}C_{λ} polynucleotides.

In the polynucleotides used in the method according to the invention, the vector further comprises a tag for purification or detection of an antibody, said tag for purification of the antibody preferably comprising a polyhistidine tail; the tag for detection of the antibody is preferably a c-*myc*-derived tag.

In another embodiment of the polynucleotides used in the method according to the invention, the vector further comprises an amber stop codon located between the second variable polynucleotide and the anchor protein.

In still another embodiment of the polynucleotides used in the method according to the invention, the vector further comprises a C_{H1} domain located between the second variable polynucleotide and the anchor protein, the C_{H1} domain preferably being a human gamma-1 C_{H1} domain.

"Anchor protein" is defined as a protein or part thereof that can at least partially be accomodated in the outer coat of a particle generated by an organism expressing the library, such as a phage or virus particle, or in the outer coat of an organism itself, in case the organism itself expresses the library. The outer coat is herein defined as the structure of a cell, virus or phage particle defining the outer surface thereof. In case of a phage or phagemid expressing the library, the anchor protein may be a coat protein, such as the gene III product. However, other systems, known to the skilled person, may be used to obtain a library according to the present invention. Therefore, e.g., transmembrane proteins, or the transmembrane domain thereof, may be contemplated to be used as anchor protein in eukaryotic expression systems. In the invention, the anchor protein may be fused to an antibody variable region or part thereof, resulting in the presentation of the said variable region to the outer environment of the organism, the region being anchored in its outer coat. In a preferred embodiment of the polynucleotides used according to the invention, the anchor protein is a minor coat protein III of a filamentous phage f_{d}. In one embodiment of the invention, the polynucleotides used in the method according to the invention, and therefore the Fab library, encodes at least 10⁹ different Fabs. In another embodiment of the invention, the Fab library encodes at least 10¹⁰ different Fabs. In still another embodiment of the invention, the Fab library encodes at least 3.7 x 10¹⁰ different Fabs. In still another embodiment of the invention, the Fab library encodes 10⁹ to 3.7 x 10¹⁰ different Fabs.

Further, the disclosure provides a Fab library, comprising
- a plurality of vectors as defined above,
   - the second cloning region in each vector forming a fusion polynucleotide encoding a plurality of fusion proteins,
- a plurality of capsid particles, wherein the plurality of vector containing the first and second pluralities of variable polynucleotides is packaged into the capsid particles, wherein
   - at least some of the capsid particles display the fusion protein encoded by the vector packaged into the capsid on the surface of the capsid.

Further the invention relates to a method of making a plurality of polynucleotides encoding a Fab library, further comprising the steps of:
- amplifying a first plurality of variable polynucleotides with a first set of primers,
- amplifying a second plurality of variable polynucleotides with a second set of primers,
   - wherein each set of primers comprises oligonucleotides designed to be homologous to the 5' and 3' end of variable polynucleotides encoding antibody variable regions or parts thereof, such that they can be used to amplify variable polynucleotide pools from natural or synthetic sources of genes while retaining all or part of the antibody's antigen combining site.

In one embodiment, the method of constructing the Fab library comprises the steps of: amplifying a plurality of variable gene pools with a set of the primers, wherein the primers comprise oligonucleotides designed to be homologous to the 5' and 3' end of variable polynucleotides encoding antibody variable regions or parts thereof, such that they can be used to amplify variable polynucleotide pools from natural or synthetic sources of genes while retaining all or part of the antibody's antigen combining site; cloning the amplified variable gene pools into a vector with a two-step procedure to obtain a Fab library; wherein the vector comprises a phage or phagemid vector which will accommodate expression of the cloned antibody variable polynucleotides as antibody Fab fragments, wherein one of the two antibody chains is fused to one of the phage coat proteins (e.g., geneIII product).

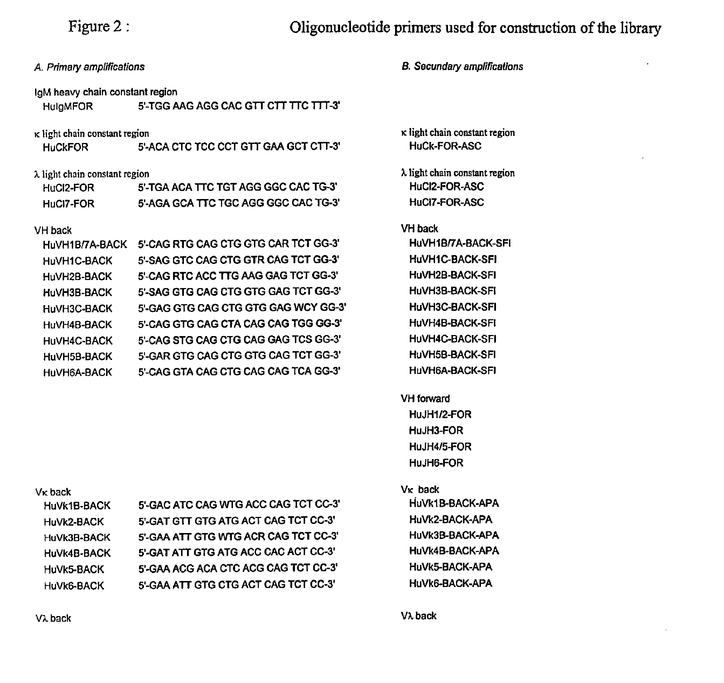

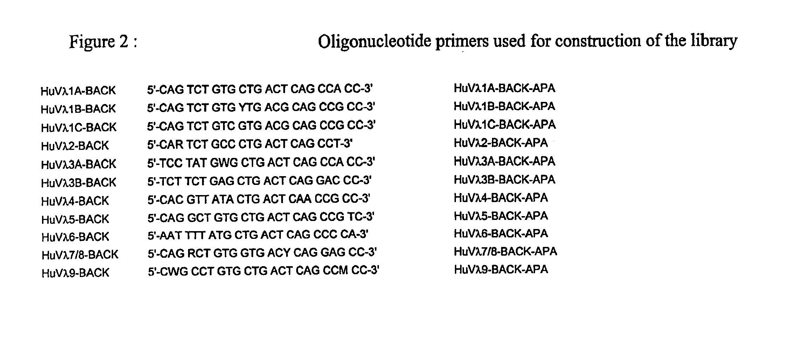

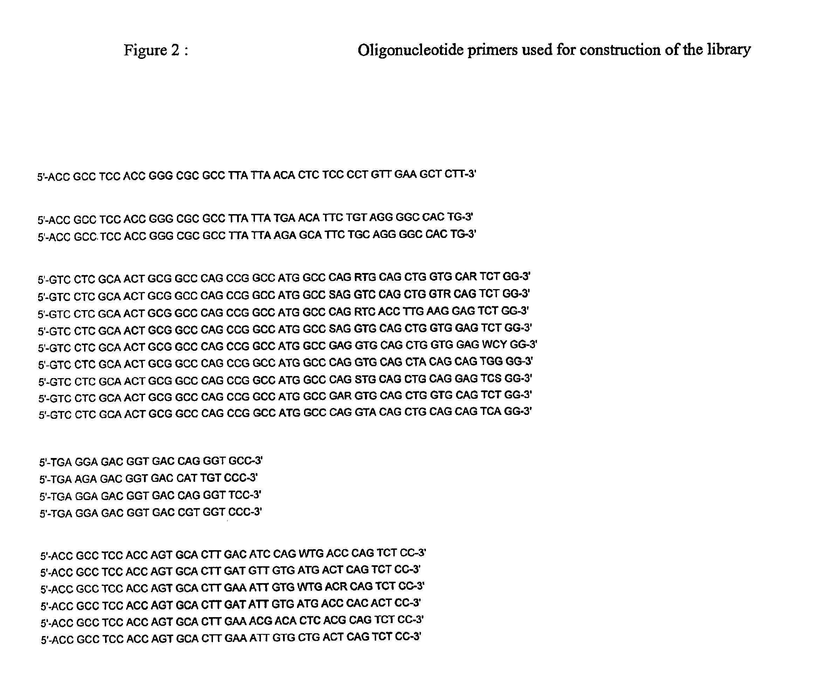

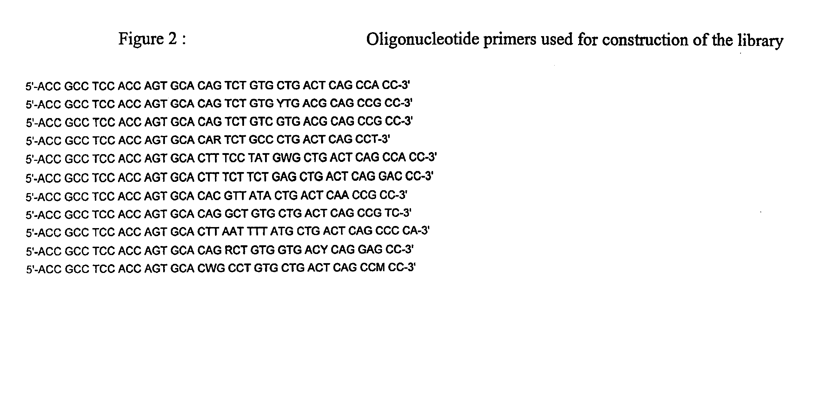

In one embodiment, the BACK primers were designed to have at the most three mutations in a total of twentyone to twentythree nucleotides when compared to the human germline gene segment region they would have to bind to, but with at least 3 homologous residues towards the 3' site of the oligonucleotide. This set of olignucleotides will recognise approximately 90% of human germline gene segments and as such provide access to most of the present diversity of the B-cells in non-immunized sources. In another embodiment, the heavy chain primers should end with 'GG' to ensure stable binding at high annealing temperatures (at least 55EC). Similarly the VκBACK primers and most of the VλBACK primers will be designed to preferentially end in 'CC'. In an alternate embodiment, the primers consist of the sequences in Figure 2.

The disclosure also relates to a vector as is defined above, comprising one of the first and one of the second plurality of the variable polynucleotides cloned into the first and second cloning region respectively.

The present disclosure further relates to host cells containing the Fab libraries obtained by the method of the invention or the polynucleotides that encode the Fab libraries.

In one aspect the method of the invention involves linking a desired specific binding pair member, such as an antibody molecule, to a phage coat protein. By then enriching for the specific binding pair member, such as by affinity techniques, for example, the DNA which encodes the specific binding pair member is also enriched and may then be isolated. The DNA so obtained may then be cloned and expressed in other systems, yielding potentially large quantities of the desired specific binding pair member, or may be subjected to sequencing and further cloning and genetic manipulations prior to expression.

Typically the target for the specific binding pair member, e.g., an antigen or hapten when the specific binding pair member is an antibody, is known, and the methods herein provide a means for creating and/or identifying a specific binding pair member which specifically binds the target of interest. Thus, when the protein is an antibody the present disclosure provides a novel means for producing antibodies, particularly monoclonal antibodies, with specificity for predetermined targets, thereby circumventing the laborious, time-consuming and often unpredictable process of conventional monoclonal antibody technology.

### Brief description of the drawings

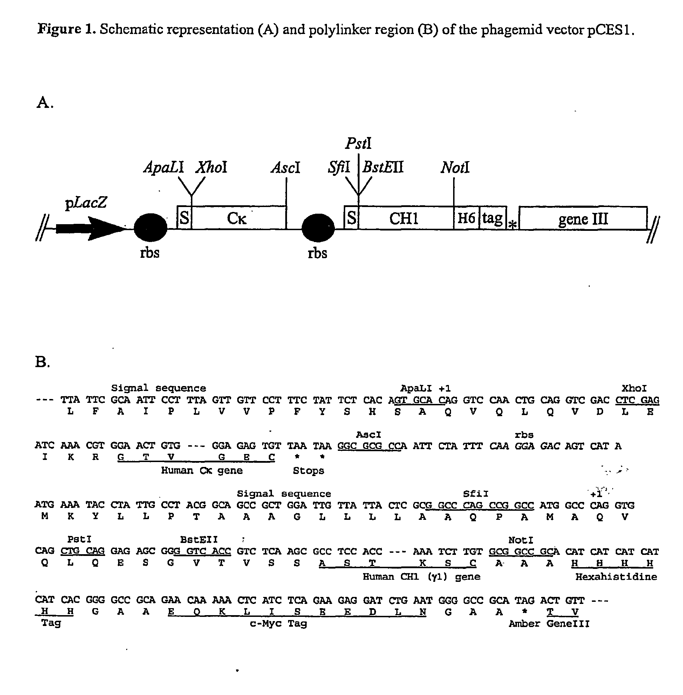

Figure 1. Phagemid vector pCES1 for display of antibody Fab fragments. Schematic representation (A) and polylinker region (B) of pCES1. The polylinker region comprises two signal sequences ("S"; pelB and the geneIII leader sequence), the Cκ domain, ribosome binding site (rbs), CH1 domain, hexa histidine tag (H6) and a c-*myc* derived sequence. Variable domain genes can be cloned as *Apa*LI *- Xho*I or *Apa*LI - *Asc* fragments (for VL or VLCL respectively) and *Sfi*I / *Pst*I *- Bst*EII or *Sfi*I - *Not*I fragments (for VH or VHCH1 respectively. The amber stop codon (*) between the antibody genes and bacteriophage gene III enables the production of soluble Fab fragments in a non-suppressor strain of *E. coli.* Expression of the bicistronic operon is under control of the LacZ promotor (p*LacZ*).
Figure 2. This figure describes oligonucleotides used in one embodiment for PCR amplification of human heavy and light chain V-regions.

The term "active" refers to those forms of the polypeptide which retain the biologic and/or immunologic activities of any naturally occurring polypeptide.

The term "activated" cells as used in this application are those which are engaged in extracellular or intracellular membrane trafficking, including the export of neurosecretory or enzymatic molecules as part of a normal or disease process.

The term "antibody" means an immunoglobulin whether natural or partly or wholly synthetically produced. The term also covers any protein or polypeptide having a binding domain which is homologous to an immunoglobulin binding domain. These proteins can be derived from natural sources, or partly or wholly synthetically produced. Example antibodies are the immunoglobulin isotypes and the Fab, scFv, Fv, dAab, VHH, Fd fragments.

The term "antibody polypeptide dimer" means an association of two polypeptide chain components of an antibody, capable of binding an antigen. Thus, it may be one arm of an antibody consisting of a heavy chain and a light chain, it may be a Fab fragment consisting of \/_{L}, V_{H}, C_{L} and C_{H1} antibody domains, or an Fv fragment consisting of a V_{L} domain and a V_{H} domain. The term "capsid" means a replicable genetic display package, with or without the genetic information. The capsids display a member of a specific binding pair at its surface. The package may be a population of bacteriophages which display an antigen binding domain, e.g., a Fab, at the surface of some or all of the capsids within the population. This type of package has been called a phage antibody (pAb).

The term "C_{H1} domain" means the first constant region of the heavy chain of an antibody or part thereoff or extended with amino acids from the hinge regions as to allow pairing of the expressed (VH)CH1 fragment with the antibody's light chain, and possible disulphide-bridge formation. This may be the CH1 domain of a human antibody of isotype gamma-1.

A "component part of an antibody antigen-binding site" may be or correspond to a polypeptide chain component, e.g., a V_{H} or a V_{L} domain. However, it may be a CDR, or a V_{L} sequence plus CDR of a V_{H}, a V_{H} sequence plus CDR of a V_{L}, a V_{H} plus V_{L} sequence lacking only a CDR, and so on. The proviso is that the first and second component parts of an antigen-binding site of an antibody must in combination (together) form an antigen-binding site. Thus, if the second component part of an antigen-binding site of a non-human antibody specific for an antigen of interest is a CDR, then the first component part of an antigen-binding site of a human antibody will comprise the remainder of a V_{H} and V_{L} region required to form a antigen-binding site (with or without associated antibody constant domains (in a Fab format), or with or without a linker peptide sequence (in a Fv format). The second component part of an antigen-binding site of a non-human antibody may comprise a V_{L} domain plus part of a V_{H} domain, that part being one or more CDRs,for instance, perhaps CDR3. In such case, the first component part of an antigen-binding site of a human antibody would comprise the remainder of a V_{H} sequence which in combination with the second component part forms an antigen-binding site. Of course, the converse situation holds and the person skilled in the art will be able to envisage other combinations of first and second component parts which together form an antigen-binding site. The term "conditionally defective" means a gene which does not express a particular polypeptide under one set of conditions, but expresses it under another set of conditions. An example, is a gene containing an amber mutation expressed in non-suppressing or suppressing hosts respectively. Alternatively, a gene may express a protein or polypeptide which is defective under one set of conditions, but not under another set. An example is a gene with a temperature sensitive mutation.

The term "derivative" refers to polypeptides chemically modified by such techniques as ubiquitination, labeling (e.g., with radionuclides or various enzymes), pegylation (derivatization with polyethylene glycol) and insertion or substitution by chemical synthesis of amino acids such as ornithine, which do not normally occur in human proteins.

The term "domain" means a part of a protein or polypeptide that is folded within itself and independently of other parts of the same protein or polypeptide and independently of a complementary binding member.

The term "eluant" means a solution used to breakdown the linkage between two molecules. The linkage can be a non-covalent or covalent bond(s). The two molecules can be members of a sbp. The term "expression modulating fragment," EMF, means a series of nucleotides which modulates the expression of an operably linked ORF or another EMF.

As used herein, a sequence is said to "modulate the expression of an operably linked sequence" when the expression of the sequence is altered by the presence of the EMF. EMFs include, but are not limited to, promoters, and promoter modulating sequences (inducible elements). One class of EMFs are fragments which induce the expression or an operably linked ORF in response to a specific regulatory factor or physiological event.

The term "Fab" refers to antibody fragments including fragments which comprise two N-terminal portions of the heavy chain polypeptide joined by at least one disulfide bridge in the hinge region and two complete light chain polypeptides, where each light chain is complexed with one N-terminal portion of a heavy chain. Fab also includes Fab fragments which comprise all or a large portion of a light chain polypeptide (e.g., V_{L}C_{L}) complexed with the N-terminal portion of a heavy chain polypeptide (e.g., V_{H}C_{H1}).

The term "Fab library" refers to a collection of Fab polynucleotide sequences within clones; or a genetically diverse collection of Fab polypeptides displayed on rgdps capable of selection or screening to provide an individual Fab polypeptide or a mixed population of Fab polypeptides. The term "folded unit" means a specific combination of an alpha-helix and/or beta-strand and/or beta-turn structure. Domains and folded units contain structures that bring together amino acids that are not adjacent in the primary structure.

The term "genetically diverse population" means antibodies or polypeptide components thereof, this is referring not only to diversity that can exist in the natural population of cells or organisms, but also diversity that can be created by artificial mutation *in vitro* or *in vivo*. Mutation *in vitro* may for example, involve random mutagenesis using oligonucleotides having random mutations of the sequence desired to be varied. *In vivo* mutagenesis may for example, use mutator strains of host microorganisms to harbour the DNA (see Example 38 of WO 92/01047). The words "unique population" may be used to denote a plurality of e.g., polypeptide chains, which are not genetically diverse i.e., they are all the same. A restricted population is one which is diverse but less so than the full repertoire of an animal. The diversity may have been reduced by prior selection, e.g.,using antigen binding specificity.

The term "helper phage" means a phage which is used to infect cells containing a defective phage genome and which functions to complement the defect. The defective phage genome can be a phagemid or a phage with some function encoding gene sequences removed. Examples of helper phages are M13KO7 M13K07 gene III no. 3; and phage displaying or encoding a binding molecule fused to a capsid protein.

The term "homologs" means polypeptides having the same or conserved residues at a corresponding position in their primary, secondary or tertiary structure. The term also extends to two or more nucleotide sequences encoding the homologous polypeptides. Example homologous peptides are the immunoglobulin isotypes.

The term "host cell" refers to a prokaryotic or eukaryotic cell into which the vectors of the method of the invention may be introduced, expressed and/or propagated. Typical prokaryotic host cells include various strains of *E. coli.* Typical eukaryotic host cells are yeast or filamentous fungi, or mammalian cells, such as Chinese hamster ovary cells, murine NIH 3t3 fibroblasts, or human embryonic kidney 193 cells.

The term "immunoglobulin superfamily" means a family of polypeptides, the members of which have at least one domain with a structure related to that of the variable or constant domain of immunoglobulin molecules. The domain contains two B-sheets and usually a conserved disulphide bond (see A. F. Williams and A. N. Barclay 1988 Ann. Rev Immunol. 6 381-405). Example members of an immunoglobulin superfamily are CD4, platelet derived growth factor receptor (PDGFR), intercellular adhesion molecule. (ICAM). Except where the context otherwise dictates, reference to immunoglobulins and immunoglobulin homologs in this application includes members of the immunoglobulin superfamily and homologs thereof.

The term "infection" refers to the introduction of nucleic acids into a suitable host cell by use of a virus or viral vector.

The term "intermediate fragment" means a nucleic acid between 5 and 1000 bases in length, and preferably between 10 and 40 bp in length.

The term "isolated" as used herein refers to a nucleic acid or polypeptide separated not only from other nucleic acids or polypeptides that are present in the natural source of the nucleic acid or polypeptide, but also from polypeptides, and preferably refers to a nucleic acid or polypeptide found in the presence of (if anything) only a solvent, buffer, ion, or other component normally present in a solution of the same. The terms "isolated" and "purified" do not encompass nucleic acids or polypeptides present in their natural source.

The term "mutator strain" means a host cell which has a genetic defect which causes DNA replicated within it to be mutated with respect to its parent DNA. Example mutator strains are NR9046mutD5 and NR9046 mut T1 (See Example 38 of WO 92/01047).

The term "naturally occurring polypeptide" refers to polypeptides produced by cells that have not been genetically engineered and specifically contemplates various polypeptides arising from post-translational modifications of the polypeptide including, but not limited to, acetylation, carboxylation, glycosylation, phosphorylation, lipidation and acylation.

The term "nucleotide sequence" refers to a heteropolymer of nucleotides or the sequence of these nucleotides. The terms "nucleic acid" and "polynucleotide" are also used interchangeably herein to refer to a heteropolymer of nucleotides. Generally, nucleic acid segments provided by this invention may be assembled from fragments of the genome and short oligonucleotide linkers, or from a series of oligonucleotides, or from individual nucleotides, to provide a synthetic nucleic acid which is capable of being expressed in a recombinant transcriptional unit comprising regulatory elements derived from a microbial or viral operon, or a eukaryotic gene.

The terms "oligonucleotide fragment" or a "polynucleotide fragment", "portion," or "segment" is a stretch of polypeptide nucleotide residues which is long enough to use in polymerase chain reaction (PCR) or various hybridization procedures to identify or amplify identical or related parts of mRNA or DNA molecules.

The terms "oligonucleotides" or "nucleic acid probes" are prepared based on the polynucleotide sequences provided in the present disclosure. Oligonucleotides comprise portions of such a polynucleotide sequence having at least about 15 nucleotides and usually at least about 20 nucleotides. Nucleic acid probes comprise portions of such a polynucleotide sequence having fewer nucleotides than about 6 kb, usually fewer than about 1 kb. After appropriate testing to eliminate false positives, these probes may, for example, be used to determine whether specific mRNA molecules are present in a cell or tissue or to isolate similar nucleic acid sequences from chromosomal DNA as described by Walsh et al. (Walsh, P.S. et al., 1992, PCR Methods Appl 1:241-250).

The term "open reading frame," ORF, means a series of nucleotide triplets coding for amino acids without any termination codons and is a sequence translatable into protein.

The term "phage vector" means a vector derived by modification of a phage genome, containing an origin of replication for a bacteriophage, but not one for a plasmid.

The term "phagemid vector" means a vector derived by modification of a plasmid genome, containing an origin of replication for a bacteriophage as well as the plasmid origin of replication. The term "phenotype" refers to a physical (e.g., pigment, or cell shape) and/or metabolic property of a cell which can be measured or exploited in some fashion and which is effected by the reporter gene.

The term "polylinker region" means a polynucleotide that contains at least two restriction enzyme sites that are unique in the vector that contains the polylinker region, i.e., these restriction sites are easily used cloning sites in the vector.

A polypeptide "fragment," "portion," or "segment" is a stretch of amino acid residues of at least about 5 amino acids, often at least about 7 amino acids, typically at least about 9 to 13 amino acids, and, in various embodiments, at least about 17 or more amino acids. To be active, any polypeptide must have sufficient length to display biologic and/or immunologic activity.

The term "probes" includes naturally occurring or recombinant or chemically synthesized single-or double-stranded nucleic acids. They may be labeled by nick translation, Klenow fill-in reaction, PCR or other methods well known in the art. Probes of the present disclosure, their preparation and/or labeling are elaborated in Sambrook, J. et al., 1989, Molecular Cloning: A Laboratory Manual, Cold Spring Harbor Laboratory, NY; or Ausubel, F.M. et al., 1989, Current Protocols in Molecular Biology, John Wiley & Sons, New York NY.

The term "purified" as used herein denotes that the indicated nucleic acid or polypeptide is present in the substantial absence of other biological macromolecules, e.g., polynucleotides, proteins, and the like. In one embodiment, the polynucleotide or polypeptide is purified such that it constitutes at least 95% by weight, more preferably at least 99.8% by weight, of the indicated biological macromolecules present (but water, buffers, and other small molecules, especially molecules having a molecular weight of less than 1000 daltons, can be present).

The term "recombinant," when used herein to refer to a polypeptide or protein, means that a polypeptide or protein is derived from recombinant (e.g., microbial or mammalian) expression systems. 'Microbial' refers to recombinant polypeptides or proteins made in bacterial or fungal (e.g., yeast) expression systems. As a product, 'recombinant microbial' defines a polypeptide or protein essentially free of native endogenous substances and unaccompanied by associated native glycosylation. Polypeptides or proteins expressed in most bacterial cultures, e.g., *E. coli*, will be free of glycosylation modifications; polypeptides or proteins expressed in yeast will have a glycosylation pattern in general different from those expressed in mammalian cells.

The term "recombinant expression vehicle or vector" refers to a plasmid or phage or virus or vector, for expressing a polypeptide from a DNA (RNA) sequence. An expression vehicle can comprise a transcriptional unit comprising an assembly of (1) a genetic element or elements having a regulatory role in gene expression, for example, promoters or enhancers, (2) a structural or coding sequence which is transcribed into mRNA and translated into protein, and (3) appropriate transcription initiation and termination sequences. Structural units intended for use in yeast or eukaryotic expression systems preferably include a leader sequence enabling extracellular secretion of translated protein by a host cell. Alternatively, where recombinant protein is expressed without a leader or transport sequence, it may include an N-terminal methionine residue. This residue may or may not be subsequently cleaved from the expressed recombinant protein or polypeptide to provide a final product.

The term "recombinant expression system" means host cells which have stably integrated a recombinant transcriptional unit into chromosomal DNA or carry the recombinant transcriptional unit extrachromosomally. Recombinant expression systems as defined herein will express heterologous polypeptides or proteins upon induction of the regulatory elements linked to the DNA segment or synthetic gene to be expressed. This term also means host cells which have stably integrated a recombinant genetic element or elements having a regulatory role in gene expression, for example, promoters or enhancers. Recombinant expression systems as defined herein will express polypeptides or proteins endogenous to the cell upon induction of the regulatory elements linked to the endogenous DNA segment or gene to be expressed. The cells can be prokaryotic or eukaryotic.

The term "recombinant variant" refers to any polypeptide differing from naturally occurring polypeptides by amino acid insertions, deletions, and substitutions, created using recombinant DNA techniques. Guidance in determining which amino acid residues may be replaced, added or deleted without abolishing activities of interest, such as cellular trafficking, may be found by comparing the sequence of the particular polypeptide with that of homologous peptides and minimizing the number of amino acid sequence changes made in regions of high homology. Preferably, amino acid "substitutions" are the result of replacing one amino acid with another amino acid having similar structural and/or chemical properties, i.e., conservative amino acid replacements. Amino acid substitutions may be made on the basis of similarity in polarity, charge, solubility, hydrophobicity, hydrophilicity, and/or the amphipathic nature of the residues involved. For example, nonpolar (hydrophobic) amino acids include alanine, leucine, isoleucine, valine, proline, phenylalanine, tryptophan, and methionine; polar neutral amino acids include glycine, serine, threonine, cysteine, tyrosine, asparagine, and glutamine; positively charged (basic) amino acids include arginine, lysine, and histidine; and negatively charged (acidic) amino acids include aspartic acid and glutamic acid.

"Insertions" or "deletions" are typically in the range of about 1 to 5 amino acids. The variation allowed may be experimentally determined by systematically making insertions, deletions, or substitutions of amino acids in a polypeptide molecule using recombinant DNA techniques and assaying the resulting recombinant variants for activity.

Alternatively, where alteration of function is desired, insertions, deletions or non-conservative alterations can be engineered to produce altered polypeptides. Such alterations can, for example, alter one or more of the biological functions or biochemical characteristics of the polypeptides. For example, such alterations may change polypeptide characteristics such as ligand-binding affinities, interchain affinities, or degradation/turnover rate. Further, such alterations can be selected so as to generate polypeptides that are better suited for expression, scale up and the like in the host cells chosen for expression. For example, cysteine residues can be deleted or substituted with another amino acid residue in order to eliminate disulfide bridges. Alternatively, recombinant variants encoding these same or similar polypeptides may be synthesized or selected by making use of the "redundancy" in the genetic code. Various codon substitutions, such as the silent changes which produce various restriction sites, may be introduced to optimize cloning into a plasmid or viral vector or expression in a particular prokaryotic or eukaryotic system. Mutations in the polynucleotide sequence may be reflected in the polypeptide or domains of other peptides added to the polypeptide to modify the properties of any part of the polypeptide, to change characteristics such as ligand-binding affinities, interchain affinities, or degradation/turnover rate.

The term "repertoire of artificially rearranged immunoglobulin genes" means a collection of nucleotide e.g., DNA, sequences derived wholly or partly from a source other than the rearranged immunoglobulin sequences from an animal. This may include for example, DNA sequences encoding VH domains by combining unrearranged V segments with D and J segments and DNA sequences encoding VL domains by combining V and J segments. Part or all of the DNA sequences may be derived by oligonucleotide synthesis.

The term "repertoire of rearranged immunoglobulin genes" means a collection of naturally occurring nucleotides e.g., DNA sequences which encoded expressed immunoglobulin genes in an animal. The sequences are generated by the *in vivo* rearrangement of e.g., V, D and J segments for H chains and e.g., the V and J segments for L chains. Alternatively the sequences may be generated from a cell line immunised *in vitro* and in which the rearrangement in response to immunisation occurs intracellularly. The word "repertoire" is used to indicate genetic diversity. The term "replicable genetic display package" (Rgdp) means a biological particle which has genetic information providing the particle with the ability to replicate. The particle can display on its surface at least part of a polypeptide. The polypeptide can be encoded by genetic information native to the particle and/or artificially placed into the particle or an ancestor of it. The displayed polypeptide may be any member of a specific binding pair e.g., heavy or light chain domains based on an immunoglobulin molecule, an enzyme or a receptor etc. The particle may be a virus e.g., a bacteriophage such as fd or M13.

The term "reporter gene" refers to a nucleic acid which encodes a protein or polypeptide that produces a phenotypic change in the host cell that may be measured and/or used to separate host cells. For example, the reporter gene may encode a protein or polypeptide that has flourescent properties, e.g., β-galactosidase, auto-fluorescent protein GFP, etc.; or the reporter gene may encode a selectable marker, e.g., antibiotic resistance; or an epitope that is expressed on the surface of the host cell.

The term "ribosome binding site" means a polyribonucleotide that allows a ribosome to select the proper initiation codon during the initiation of translation. In some prokaryotes, this polyribonucleotide is called the Shine-Dalgarno sequence, and the Shine-Delgarno sequence base pairs with the 16S RNA of the ribosome.

The term "secreted" protein or polypeptide refers to a protein or polypeptide that is transported across or through a membrane, including transport as a result of signal sequences in its amino acid sequence when it is expressed in a suitable host cell. "Secreted" proteins or polypeptides include without limitation proteins or polypeptides secreted wholly (e.g., soluble proteins) or partially (e.g., receptors) from the cell in which they are expressed. "Secreted" proteins or polypeptides also include without limitation proteins or polypeptides which are transported across the membrane of the endoplasmic reticulum.

The term "signal sequence" means an amino acid sequence that is found at the amino terminus of a polypeptide and directs transportation of the polypeptide across or through a membrane. Signal sequences include amino terminal polypeptides that are 13-36 residues long, and have a 7 to 13 residue hydrophobic core flanked by several hydrophilic residues that usually include one or more basic residues near the N-terminus.

The term "stringent" is used to refer to conditions that are commonly understood in the art as stringent. An exemplary set of conditions include a temperature of 60-70°C, (preferably about 65°C) and a salt concentration of 0.70 M to 0.80 M (preferably about 0.75M). Further exemplary conditions include, hybridizing conditions that (1) employ low ionic strength and high temperature for washing, for example, 0.015 M NaCl/0.0015 M sodium citrate/0.1% SDS at 50°C; (2) employ during hybridization a denaturing agent such as formamide, for example, 50% (vol/vol) formamide with 0.1 % bovine serum albumin/0.1 % Ficoll/0.1% polyvinylpyrrolidone/50 mM sodium phosphate buffer at pH 6.5 with 750 mM NaCl, 75 mM sodium citrate at 42°C; or (3) employ 50% formamide, 5 x SSC (0.75 M NaCl, 0.075 M Sodium pyrophosphate, 5 x Denhardt's solution, sonicated salmon sperm DNA (50 g/ml), 0.1% SDS, and 10% dextran sulfate at 42°C, with washes at 42°C in 0.2 x SSC and 0.1 % SDS.

In instances wherein hybridization of deoxyoligonucleotides is concerned, additional exemplary stringent hybridization conditions include washing in 6xSSC/0.05% sodium pyrophosphate at 37°C (for 14-base oligos), 48°C (for 17-base oligos), 55°C (for 20-base oligos), and 60°C (for 23-base oligos).

As used herein, "substantially equivalent" can refer both to nucleotide and amino acid sequences, for example a mutant sequence, that varies from a reference sequence by one or more substitutions, deletions, or additions, the net effect of which does not result in an adverse functional dissimilarity between the reference and subject sequences. Typically, such a substantially equivalent sequence varies from one of those listed herein by no more than about 20% (i.e., the number of individual residue substitutions, additions, and/or deletions in a substantially equivalent sequence, as compared to the corresponding reference sequence, divided by the total number of residues in the substantially equivalent sequence is about 0.2 or less). Such a sequence is said to have 80% sequence identity to the listed sequence. In one embodiment, a substantially equivalent, e.g., mutant, sequence of the disclosure varies from a listed sequence by no more than 10% (90% sequence identity); in a variation of this embodiment, by no more than 5% (95% sequence identity); and in a further variation of this embodiment, by no more than 2% (98% sequence identity). Substantially equivalent, e.g., mutant, amino acid sequences according to the disclosure generally have at least 95% sequence identity with a listed amino acid sequence, whereas substantially equivalent nucleotide sequence of the disclosure can have lower percent sequence identities, taking into account, for example, the redundancy or degeneracy of the genetic code. For the purposes of the present disclosure, sequences having substantially equivalent biological activity and substantially equivalent expression characteristics are considered substantially equivalent. For the purposes of determining equivalence, truncation of the mature sequence (e.g., via a mutation which creates a spurious stop codon) should be disregarded.

Nucleic acid sequences encoding such substantially equivalent sequences, e.g., sequences of the recited percent identities, can routinely be isolated and identified via standard hybridization procedures well known to those of skill in the art.

The term "suppressible translational stop codon" means a codon which allows the translation of nucleotide sequences downstream of the codon under one set of conditions, but under another set of conditions translation ends at the codon. Examples of suppressible translational stop codons are the amber, ochre and opal codons.

The term "tag" means an extension of the antibody Fab fragment, for example expressed at the carboxyterminus of the heavy chain, that comprises at least one amino acids but more typically five to fifteen amino acids, and that can be specifically recognised by an antibody or other binding ligand or binding matrix for the sequence. Tags may be combined in the same Fab. Examples are a stretch of five histidine residues that can be recognised by specific antibodies and by defined immobilised metal ions, and a stretch of the following 12 amino acids (EQKLISEEDLN) that are recognised by the 9E10 antibody (Marks et al., 1991).

The term "target" means any molecule that is antigenic, e.g., can be recognized with reasonably specificity by an antibody from the Fab library.

The term "target element" refers to a nucleic acid sequence that alters the expression of the target gene. Target elements include, but are not limited to, promoters, and promoter modulating sequences (inducible elements). One class of target elements are fragments which induce the expression in response to a specific regulatory factor or physiological event.

The term "transfection" refers to the taking up of an expression vector by a suitable host cell, whether or not any coding sequences are in fact expressed.

The term "transformation" means introducing DNA into a suitable host cell so that the DNA is replicable, either as an extrachromosomal element, or by chromosomal integration.

The term "universal set" refers to a set of nucleic acids, most preferably a set of oligonucleotides, which represent all possible combinations of sequence for a given length of nucleotides, e.g., all 4096 insert oligonucleotides six nucleotides in length. In a preferred embodiment, the term universal set refers to the set of all possible oligonucleotides of a given length, wherein one or more positions in the oligonucleotides are held constant (i.e., the same nucleotide is present at this position in all members of the set).

As used herein, an "uptake modulating fragment," UMF, means a series of nucleotides which mediate the uptake of a linked DNA fragment into a cell. UMFs can be readily identified using known UMFs as a target sequence or target motif with the computer-based systems described below.

The presence and activity of a UMF can be confirmed by attaching the suspected UMF to a marker sequence. The resulting nucleic acid molecule is then incubated with an appropriate host under appropriate conditions and the uptake of the marker sequence is determined. As described above, a UMF will increase the frequency of uptake of a linked marker sequence.

The term "vector" refers to a plasmid or phage or virus or vector, for expressing a polypeptide from a DNA (RNA) sequence. The vector can comprise a transcriptional unit comprising an assembly of (1) a genetic element or elements having a regulatory role in gene expression, for example, promoters or enhancers, (2) a structural or coding sequence which is transcribed into mRNA and translated into protein, and (3) appropriate translation initiation and termination sequences. Structural units intended for use in yeast or eukaryotic expression systems may include a leader sequence enabling extracellular secretion of translated protein by a host cell. The term "V_{L} polynucleotides" means polynucleotides encoding the CDR containing domains of some or all of the light chain genes from the V_{κ} and/or V_{λ}- families.

The term "V_{H} polynucleotides" means polynucleotides encoding the CDR containing domains of some or all of the heavy chain genes from the heavy chain gene family.

Each of the above terms is meant to encompasses all that is described for each, unless the context dictates otherwise.

The recombinant constructs of the present disclosure comprise a vector, such as a plasmid or viral vector, into which a nucleic acid(s) of interest may be inserted. The vector may further comprise regulatory sequences, including for example, a promoter, operably linked to the nucleic acid(s) of interest. Large numbers of suitable vectors and promoters are known to those of skill in the art and are commercially available for generating the recombinant constructs of the method of the present invention. The following vectors are provided by way of example. Bacterial: pBs, phagescript, PsiX174, pBluescript SK, pBs KS, pNH8a, pNH16a, pNH18a, pNH46a (Stratagene); pTrc99A, pKK223-3, pKK233-3, pDR540, pRIT5 (Pharmacia). Eukaryotic: pWLneo, pSV2cat, pOG44, PXTI, pSG (Stratagene) pSVK3, pBPV, pMSG, pSVL (Pharmacia).

Methods which are well known to those skilled in the art can be used to construct vectors containing a polynucleotide used in the method of the invention and appropriate transcriptional/translational control signals. These methods include *in vitro* recombinant DNA techniques, synthetic techniques and *in vivo* recombination/genetic recombination. See, for example, the techniques described in Maniatis et al., Molecular Cloning A Laboratory Manual, Cold Spring Harbor Laboratory, N.Y. (1989) and Ausubel et al., Current Protocols in Molecular Biology, Greene Publishing Associates and Wiley Interscience, N.Y. (1989).

Promoter regions can be selected from any desired gene using CAT (chloramphenicol transferase) vectors or other vectors with selectable markers. Two appropriate vectors are pKK232-8 and pCM7. Particular named bacterial promoters include lacl, lacZ, T3, T7, gpt, lambda P, and trc. Eukaryotic promoters include CMV immediate early, HSV thymidine kinase, early and late SV40, LTRs from retrovirus, and mouse metallothionein-I.

Generally, recombinant expression vectors will include origins of replication and selectable markers permitting transformation of the host cell, e.g., the ampicillin resistance gene of *E. coli* and *S*. *cerevisiae* TRP1 gene, and a promoter derived from a highly-expressed gene to direct transcription of a downstream structural sequence. Such promoters can be derived from operons encoding glycolytic enzymes such as 3-phosphoglycerate kinase (PGK), a-factor, acid phosphatase, or heat shock proteins, among others. The polynucleotide used in the method of the invention is assembled in appropriate phase with translation initiation and termination sequences, and preferably, a leader sequence capable of directing secretion of translated protein into the periplasmic space or extracellular medium. Optionally, the polynucleotide used in the method of the invention can encode a fusion protein including an N-terminal identification peptide imparting desired characteristics, e.g., stabilization or simplified purification of expressed recombinant product.

Useful expression vectors for bacteria are constructed by inserting a polynucleotide used in the method of the invention together with suitable translation initiation and termination signals, optionally in operable reading phase with a functional promoter. The vector will comprise one or more phenotypic selectable markers and an origin of replication to ensure maintenance of the vector and to, if desirable, provide amplification within the host. Suitable prokaryotic hosts for transformation include *E. coli*, *Bacillus subtilis*, *Salmonella typhimurium* and various species within the genera Pseudomonas, Streptomyces, and Staphylococcus, although others may also be employed as a matter of choice.

As a representative but nonlimiting example, useful expression vectors for bacteria can comprise a selectable marker and bacterial origin of replication derived from commercially available plasmids comprising genetic elements of the well known cloning vector pBR322 (ATCC 37017). Such commercial vectors include, for example, pKK223-3 (Pharmacia Fine Chemicals, Uppsala, Sweden) and GEM 1 (Promega Biotec, Madison, WI, USA). These pBR322 "backbone" sections are combined with an appropriate promoter and the structural sequence to be expressed. The present disclosure further provides host cells containing the vectors of the presentdisclosure, wherein the nucleic acid has been introduced into the host cell using known transformation, transfection or infection methods. The host cell can be a higher eukaryotic host cell, such as a mammalian cell, a lower eukaryotic host cell, such as a yeast cell, or the host cell can be a prokaryotic cell, such as a bacterial cell. Introduction of the recombinant construct into the host cell can be effected, for example, by calcium phosphate transfection, DEAE, dextran mediated transfection, or electroporation (Davis, L. et al., Basic Methods in Molecular Biology (1986)). Any host/vector system can be used to identify one or more of the target elements of the presentdisclosure. These include, but are not limited to, eukaryotic hosts such as HeLa cells, Cv-1 cell, COS cells, and Sf9 cells, as well as prokaryotic host such as *E. coli* and *B*. *subtilis.* The most preferred cells are those which do not normally express the particular reporter polypeptide or protein or which expresses the reporter polypeptide or protein at low natural level.

The host may also be a yeast or other fungi. In yeast, a number of vectors containing constitutive or inducible promoters may be used. For a review see, Current Protocols in Molecular Biology, Vol. 2, Ed. Ausubel et al., Greene Publish. Assoc. & Wiley Interscience, Ch. 13 (1988); Grant et al., Expression and Secretion Vectors for Yeast, in Methods in Enzymology, Ed. Wu & Grossman, Acad. Press, N.Y. 153:516-544 (1987); Glover, DNA Cloning, Vol. II, IRL Press, Wash., D.C., Ch. 3 (1986); Bitter, Heterologous Gene Expression in Yeast, in Methods in Enzymology, Eds. Berger & Kimmel, Acad. Press, N.Y. 152:673-684 (1987); and The Molecular Biology of the Yeast Saccharomyces, Eds. Strathern et al., Cold Spring Harbor Press, Vols. I and II (1982).

The host may also be a prokaryotic cell such as *E. coli,* other enterobacteriaceae such as *Serratia marescans*, bacilli, various pseudomonads, or other prokaryotes which can be transformed, transfected, infected, etc. (i.e., a method exists for introducing nucleic acids to the host cell).

The present disclosure further provides host cells genetically engineered to contain the polynucleotides used in the method of the invention. For example, such host cells may contain nucleic acids of the disclosure introduced into the host cell using known transformation, transfection or infection methods. The present disclosure still further provides host cells genetically engineered to express the polynucleotides of thedisclosure, wherein such polynucleotides are in operative association with a regulatory sequence heterologous to the host cell which drives expression of the polynucleotides in the cell.

The host cell can be a higher eukaryotic host cell, such as a mammalian cell, a lower eukaryotic host cell, such as a yeast cell, or the host cell can be a prokaryotic cell, such as a bacterial cell. Introduction of the recombinant construct into the host cell can be effected by calcium phosphate transfection, DEAE, dextran mediated transfection, or electroporation (Davis, L. et al., Basic Methods in Molecular Biology (1986)). The host cells containing one of polynucleotides of the disclosure, can be used in conventional manners to produce the gene product encoded by the isolated fragment (in the case of an ORF) or can be used to produce a heterologous protein under the control of the EMF.

Any host/vector system can be used to express one or more of the ORFs of the present disclosure. These include, but are not limited to, eukaryotic hosts such as HeLa cells, Cv-1 cell, COS cells, and Sf9 cells, as well as prokaryotic host such as *E. coli* and *B. subtilis.* The most preferred cells are those which do not normally express the particular polypeptide or protein or which expresses the polypeptide or protein at low natural level. Mature proteins can be expressed in mammalian cells, yeast, bacteria, or other cells under the control of appropriate promoters. Cell-free translation systems can also be employed to produce such proteins using RNAs derived from the DNA constructs of the presentdisclosure. Appropriate cloning and expression vectors for use with prokaryotic and eukaryotic hosts are described by Sambrook, et al., in Molecular Cloning: A Laboratory Manual, Second Edition, Cold Spring Harbor, New York (1989).

Various mammalian cell culture systems can also be employed to express recombinant protein. Examples of mammalian expression systems include the COS-7 lines of monkey kidney fibroblasts, described by Gluzman, Cell 23:175 (1981), and other cell lines capable of expressing a compatible vector, for example, the C127, 3T3, CHO, HeLa and BHK cell tines. Mammalian expression vectors will comprise an origin of replication, a suitable promoter and also any necessary ribosome binding sites, polyadenylation site, splice donor and acceptor sites, transcriptional termination sequences, and 5' flanking nontranscribed sequences. DNA sequences derived from the SV40 viral genome, for example, SV40 origin, early promoter, enhancer, splice, and polyadenylation sites may be used to provide the required nontranscribed genetic elements. Recombinant polypeptides and proteins produced in bacterial culture are usually isolated by initial extraction from cell pellets, followed by one or more salting-out, aqueous ion exchange or size exclusion chromatography steps. Protein refolding steps can be used, as necessary, in completing configuration of the mature protein. Finally, high performance liquid chromatography (HPLC) can be employed for final purification steps. Microbial cells employed in expression of proteins can be disrupted by any convenient method, including freeze-thaw cycling, sonication, mechanical disruption, or use of cell lysing agents.

A number of types of cells may act as suitable host cells for expression of the protein. Mammalian host cells include, for example, monkey COS cells, Chinese Hamster Ovary (CHO) cells, human kidney 293 cells, human epidermal A431 cells, human Colo205 cells, 3T3 cells, CV-1 cells, other transformed primate cell lines, normal diploid cells, cell strains derived from *in vitro* culture of primary tissue, primary explants, HeLa cells, mouse L cells, BHK, HL-60, U937, HaK or Jurkat cells.

Alternatively, it may be possible to produce the protein in lower eukaryotes such as yeast or in prokaryotes such as bacteria. Potentially suitable yeast strains include Saccharomyces cerevisiae, Schizosaccharomyces pombe, Kluyveromyces strains, Candida, or any yeast strain capable of expressing heterologous proteins. Potentially suitable bacterial strains include Escherichia coli, Bacillus subtilis, Salmonella typhimurium, or any bacterial strain capable of expressing heterologous proteins. If the protein is made in yeast or bacteria, it may be necessary to modify the protein produced therein, for example by phosphorylation or glycosylation of the appropriate sites, in order to obtain the functional protein. Such covalent attachments may be accomplished using known chemical or enzymatic methods.

Cells and tissues may be engineered to express an endogenous gene comprising the polynucleotides of the disclosure under the control of inducible regulatory elements, in which case the regulatory sequences of the endogenous gene may be replaced by homologous recombination. As described herein, gene targeting can be used to replace a gene's existing regulatory region with a regulatory sequence isolated from a different gene or a novel regulatory sequence synthesized by genetic engineering methods. Such regulatory sequences may be comprised of promoters, enhancers, scaffold-attachment regions, negative regulatory elements, transcriptional initiation sites, regulatory protein binding sites or combinations of said sequences. Alternatively, sequences which affect the structure or stability of the RNA or protein produced may be replaced, removed, added, or otherwise modified by targeting, including polyadenylation signals. mRNA stability elements, splice sites, leader sequences for enhancing or modifying transport or secretion properties of the protein, or other sequences which alter or improve the function or stability of protein or RNA molecules.

The targeting event may be a simple insertion of the regulatory sequence, placing the gene under the control of the new regulatory sequence, e.g., inserting a new promoter or enhancer or both upstream of a gene. Alternatively, the targeting event may be a simple deletion of a regulatory element, such as the deletion of a tissue-specific negative regulatory element. Alternatively, the targeting event may replace an existing element; for example, a tissue-specific enhancer can be replaced by an enhancer that has broader or different cell-type specificity than the naturally occurring elements. Here, the naturally occurring sequences are deleted and new sequences are added. In all cases, the identification of the targeting event may be facilitated by the use of one or more selectable marker genes that are contiguous with the targeting DNA, allowing for the selection of cells in which the exogenous DNA has integrated into the host cell genome. The identification of the targeting event may also be facilitated by the use of one or more marker genes exhibiting the property of negative selection, such that the negatively selectable marker is linked to the exogenous DNA, but configured such that the negatively selectable marker flanks the targeting sequence, and such that a correct homologous recombination event with sequences in the host cell genome does not result in the stable integration of the negatively selectable marker. Markers useful for this purpose include the Herpes Simplex Virus thymidine kinase (TK) gene or the bacterial xanthine-guanine phosphoribosyl-transferase (gpt) gene.

The gene targeting or gene activation techniques which can be used in accordance with this aspect of the invention are more particularly described in U.S. Patent No. 5,272,071 to Chappel; U.S. Patent No. 5,578,461 to Sherwin et al.; WO 93/09222 by Selden et al.; and WO 91/06667 by Skoultchi et al.

In general, techniques for preparing polyclonal and monoclonal antibodies as well as hybridomas capable of producing the desired antibody are well known in the art (Campbell, A.M., Monoclonal Antibodies Technology: Laboratory Techniques in Biochemistry and Molecular Biology, Elsevier Science Publishers, Amsterdam, The Netherlands (1984); St. Groth et al., J. Immunol. 35:1-21 (1990); Kohler and Milstein, Nature 256:495-497 (1975)), the trioma technique, the human B-cell hybridoma technique (Kozbor et al., Immunology Today 4:72 (1983); Cole et al., in Monoclonal Antibodies and Cancer Therapy, Alan R. Liss, Inc. (1985), pp. 77-96).

Methods for immunization are well known in the art. Such methods include subcutaneous or interperitoneal injection of the polypeptide. One skilled in the art will recognize that the amount of the protein encoded by the reporter gene used for immunization will vary based on the animal which is immunized, the antigenicity of the peptide and the site of injection.

The polypeptide or protein of the disclosure which is used as an immunogen may be modified or administered in an adjuvant in order to increase the polypeptide or protein's antigenicity. Methods of increasing the antigenicity of a polypeptide or protein are well known in the art and include, but are not limited to, coupling the antigen with a heterologous protein (such as globulin or β-galactosidase) or through the inclusion of an adjuvant during immunization.

For monoclonal antibodies, spleen cells from the immunized animals are removed, fused with myeloma cells, such as SP2/0-Ag14 myeloma cells, and allowed to become monoclonal antibody producing hybridoma cells.

Any one of a number of methods well known in the art can be used to identify the hybridoma cell which produces an antibody with the desired characteristics. These include screening the hybridomas with an ELISA assay, western blot analysis, or radioimmunoassay (Lutz et al., Exp. Cell Research. 175:109-124 (1988)).

Hybridomas secreting the desired antibodies are cloned and the class and subclass is determined using procedures known in the art (Campbell, A.M., Monoclonal Antibody Technology: Laboratory Techniques in Biochemistry and Molecular Biology, Elsevier Science Publishers, Amsterdam, The Netherlands (1984)).

For polyclonal antibodies, antibody containing antisera is isolated from the immunized animal and is screened for the presence of antibodies with the desired specificity using one of the above-described procedures.

Host cells are transfected or preferably infected or transformed with the above-described vectors, and cultured in nutrient media appropriate for selecting transductants ortransformants containing the vector.

The host cells which express the polypeptide or protein of the disclosure product may be identified by at least four general approaches; (a) DNA-DNA or DNA-RNA hybridization; (b) the presence or absence of gene functions; (c) assessing the level of transcription as measured by the expression of mRNA transcripts in the host cell; and (d) detection of the gene product as measured by immunoassay or by its biological activity.

In the first approach, the presence of the polypeptide or protein of the disclosure inserted in the vector can be detected by DNA-DNA or DNA-RNA hybridization using probes comprising nucleotide sequences that are homologous to the polypeptide or protein of the disclosure, respectively, or portions or derivatives thereof.

In the second approach, the recombinant expression vector/host system can be identified and selected based upon the presence or absence of certain "marker" gene functions (e.g., thymidine kinase activity, resistance to antibiotics, resistance to methotrexate, transformation phenotype, occlusion body formation in baculovirus, etc.). For example, if the polypeptide or protein of the disclosure is inserted within a marker gene sequence of the vector, recombinant cells containing the polypeptide or protein of the disclosure can be identified by the absence of the marker gene function. Alternatively, a marker gene can be placed in tandem with the polypeptide or protein of the disclosure under the control of the same or different promoter used to control the expression of the polypeptide or protein of r the disclosure. Z

Expression of the marker in response to induction or selection indicates expression of the polypeptide or protein of the disclosure.

In the third approach, transcriptional activity of the polypeptide or protein of the disclosure can be assessed by hybridization assays. For example, RNA can be isolated and analyzed by Northern blot using a probe homologous to the polypeptide or protein of the disclosure or particular portions thereof. Alternatively, total nucleic acids of the host cell may be extracted and assayed for hybridization to such probes.

In the fourth approach, the expression of a product from the polypeptide or protein of the disclosure can be assessed immunologically, for example by Western blots, immunoassays such as radioimmuno-precipitation, enzyme-linked immunoassays and the like.

A polynucleotide according to the disclosure can be joined to any of a variety of other nucleotide sequences by well-established recombinant DNA techniques (see Sambrook J et al. (1989) Molecular Cloning: A Laboratory Manual, Cold Spring Harbor Laboratory, NY). Useful nucleotide sequences for joining to polypeptides include an assortment of vectors, e.g., plasmids, cosmids, lambda phage derivatives, phagemids, and the like, that are well known in the art. Accordingly, also provided is a vector including a polynucleotide of the disclosure and a host cell containing the polynucleotide. In general, the vector contains an origin of replication functional in at least one organism, convenient restriction endonuclease sites, and a selectable marker for the host cell. Vectors according to the disclosure include expression vectors, replication vectors, probe generation vectors, and sequencing vectors. A host cell according to the disclosure can be a prokaryotic or eukaryotic cell and can be a unicellular organism or part of a multicellular organism.

The present disclosure further provides recombinant constructs comprising a nucleic acid of the disclosure, or a fragment thereof. The recombinant constructs of the present disclosure comprise a vector, such as a plasmid or viral vector, into which a nucleic acid of the disclosure, or a fragment thereof is inserted, in a forward or reverse orientation. In the case of a vector comprising one of the ORFs of the present disclosure, the vector may further comprise regulatory sequences, including for example, a promoter, operably linked to the ORF. For vectors comprising the EMFs and UMFs of the present disclosure, the vector may further comprise a marker sequence or heterologous ORF operably linked to the EMF or UMF. Large numbers of suitable vectors and promoters are known to those of skill in the art and are commercially available for generating the recombinant constructs of the present disclosure. The following vectors are provided by way of example. Bacterial: pBs, phagescript, PsiX174, pBluescript SK, pBs KS, pNH8a, pNH16a, pNH18a, pNH46a (Stratagene); pTrc99A, pKK223-3, pKK233-3, pDR540, pRIT5 (Pharmacia). Eukaryotic: pWLneo, pSV2cat, pOG44, PXTI, pSG (Stratagene) pSVK3, pBPV, pMSG, pSVL (Pharmacia).

The isolated polynucleotide of the disclosure may be operably linked to an expression control sequence such as the pMT2 or pED expression vectors disclosed in Kaufman et al., Nucleic Acids Res. 19, 4485-4490 (1991), in order to produce the protein or polypeptide recombinantly. Many suitable expression control sequences are known in the art. General methods of expressing recombinant proteins are also known and are exemplified in R. Kaufman, Methods in Enzymology 185, 537-566 (1990). As defined herein "operably linked" means that the isolated polynucleotide and an expression control sequence are situated within a vector or cell in such a way that the protein or polypeptide is expressed by a host cell which has been transformed (transfected) with the ligated polynucleotide/expression control sequence.

Promoter regions can be selected from any desired gene using CAT (chloramphenicol transferase) vectors or other vectors with selectable markers. Two appropriate vectors are pKK232-8 and pCM7. Particular named bacterial promoters include lacI, lacZ, T3, T7, gpt, lambda P_{R}, and trc. Eukaryotic promoters include CMV immediate early, HSV thymidine kinase, early and late SV40, LTRs from retrovirus, and mouse metallothionein-I. Selection of the appropriate vector and promoter is well within the level of ordinary skill in the art. Generally, recombinant expression vectors will include origins of replication and selectable markers permitting transformation of the host cell, e.g., the ampicillin resistance gene of *E. coli* and *S*. *cerevisiae* TRP1 gene, and a promoter derived from a highly-expressed gene to direct transcription of a downstream structural sequence. Such promoters can be derived from operons encoding glycolytic enzymes such as 3-phosphoglycerate kinase (PGK), a-factor, acid phosphatase, or heat shock proteins, among others. The heterologous structural sequence is assembled in appropriate phase with translation initiation and termination sequences, and preferably, a leader sequence capable of directing secretion of translated protein or polypeptide into the periplasmic space or extracellular medium. Optionally, the heterologous sequence can encode a fusion protein including an N-terminal identification peptide imparting desired characteristics, e.g., stabilization or simplified purification of expressed recombinant product. Useful expression vectors for bacterial use are constructed by inserting a structural DNA sequence encoding a desired protein or polypeptide together with suitable translation initiation and termination signals in operable reading phase with a functional promoter. The vector will comprise one or more phenotypic selectable markers and an origin of replication to ensure maintenance of the vector and to, if desirable, provide amplification within the host. Suitable prokaryotic hosts for transformation include *E*. *coli*, *Bacillus subtilis, Salmonella typhimurium* and various species within the genera Pseudomonas, Streptomyces, and Staphylococcus, although others may also be employed as a matter of choice.

As a representative but non-limiting example, useful expression vectors for bacterial use can comprise a selectable marker and bacterial origin of replication derived from commercially available plasmids comprising genetic elements of the well known cloning vector pBR322 (ATCC 37017). Such commercial vectors include, for example, pKK223-3 (Pharmacia Fine Chemicals, Uppsala, Sweden) and GEM 1 (Promega Biotec, Madison, WI, USA). These pBR322 'backbone' sections are combined with an appropriate promoter and the structural sequence to be expressed. Following transformation of a suitable host strain and growth of the host strain to an appropriate cell density, the selected promoter is induced or derepressed by appropriate means (e.g., temperature shift or chemical induction) and cells are cultured for an additional period. Cells are typically harvested by centrifugation, disrupted by physical or chemical means, and the resulting crude extract retained for further purification.

The polynucleotides of the disclosure are further directed to sequences which encode variants of the polypeptides or proteins of the disclosure. These amino acid sequence variants may be prepared by methods known in the art by introducing appropriate nucleotide changes into a native or variant polynucleotide. There are two variables in the construction of amino acid sequence variants: the location of the mutation and the nature of the mutation. The amino acid sequence variants of the nucleic acids are preferably constructed by mutating the polynucleotide to give an amino acid sequence that does not occur in nature. These amino acid alterations can be made at sites that differ in the nucleic acids from different species (variable positions) or in highly conserved regions (constant regions). Sites at such locations will typically be modified in series, e.g., by substituting first with conservative choices (e.g., hydrophobic amino acid to a different hydrophobic amino acid) and then with more distant choices (e.g., hydrophobic amino acid to a charged amino acid), and then deletions or insertions may be made at the target site. Amino acid sequence deletions generally range from about 1 to 30 residues, preferably about 1 to 10 residues, and are typically contiguous. Amino acid insertions include amino- and/or carboxyl-terminal fusions ranging in length from one to one hundred or more residues, as well as intrasequence insertions of single or multiple amino acid residues. Intrasequence insertions may range generally from about 1 to 10 amino residues, preferably from 1 to 5 residues. Examples of terminal insertions include the heterologous signal sequences necessary for secretion or for intracellular targeting in different host cells.

Amino acid sequence deletions generally range from about 1 to 30 residues, preferably about 1 to 10 residues, and are typically contiguous. Amino acid insertions include amino- and/or carboxyl-terminal fusions ranging in length from one to one hundred or more residues, as well as intrasequence insertions of single or multiple amino acid residues. Intrasequence insertions may range generally from about 1 to 10 amino residues, preferably from 1 to 5 residues. Examples of terminal insertions include the heterologous signal sequences necessary for secretion or for intracellular targeting in different host cells.

PCR may also be used to create amino acid sequence variants of the polynucleotides of the disclosure. When small amounts of template DNA are used as starting material, primer(s) that differs slightly in sequence from the corresponding region in the template DNA can generate the desired amino acid variant. PCR amplification results in a population of product DNA fragments that differ from the polynucleotide template encoding the polypeptide or protein at the position specified by the primer. The product DNA fragments replace the corresponding region in the plasmid and this gives the desired amino acid variant.

In a preferred method, polynucleotides encoding the polynucleotides of the disclosure are changed via site-directed mutagenesis. This method uses oligonucleotide sequences that encode the polynucleotide sequence of the desired amino acid variant, as well as a sufficient adjacent nucleotide on both sides of the changed amino acid to form a stable duplex on either side of the site of being changed. In general, the techniques of site-directed mutagenesis are well known to those of skill in the art and this technique is exemplified by publications such as, Edelman et al., DNA 2:183 (1983). A versatile and efficient method for producing site-specific changes in a polynucleotide sequence was published by Zoller and Smith, Nucleic Acids Res. 10:6487-6500 (1982). PCR may also be used to create amino acid sequence variants of the novel nucleic acids. When small amounts of template DNA are used as starting material, primer(s) that differs slightly in sequence from the corresponding region in the template DNA can generate the desired amino acid variant. PCR amplification results in a population of product DNA fragments that differ from the polynucleotide template encoding the polypeptide at the position specified by the primer. The product DNA fragments replace the corresponding region in the plasmid and this gives the desired amino acid variant.

A further technique for generating amino acid variants is the cassette mutagenesis technique described in Wells et al., Gene 34:315 (1985); and other mutagenesis techniques well known in the art, such as, for example, the techniques in Sambrook *et al*., supra, and Current Protocols in Molecular Biolog, Ausubel *et al*.

The present disclosure also provides both full-length and mature forms of the polypeptides or proteins of the disclosure. The full-length form of the such polypeptides or proteins can be identified by translation of the nucleotide sequence of each polynucleotide of the disclosure. The mature form of such polypeptide or protein may be obtained by expression of the full-length polynucleotide in a suitable mammalian cell or other host cell. The sequence of the mature form of the polypeptide or protein may also be determinable from the amino acid sequence of the full-length form.

Where the protein or polypeptide of the present disclosure is membrane-bound (e.g., is a receptor), the present disclosure also provides for soluble forms of such protein or polypeptide. In such forms part or all of the intracellular and transmembrane domains of the protein or polypeptide are deleted such that the protein or polypeptide is fully secreted from the cell in which it is expressed. The intracellular and transmembrane domains of proteins or polypeptides of the disclosure can be identified in accordance with known techniques for determination of such domains from sequence information.

The disclosure also relates to methods for producing a polypeptide or protein of the disclosure comprising growing a culture of the cells of the disclosure in a suitable culture medium, and purifying the protein or polypeptide of the disclosure from the culture. For example, the methods of the invention include a process for producing a polypeptide or protein of the disclosure in which a host cell containing a suitable expression vector that includes a polynucleotide or protein of the disclosure is cultured under conditions that allow expression of the encoded polypeptide or protein. The polypeptide or protein can be recovered from the culture, conveniently from the culture medium, and further purified.

The present disclosure further provides isolated polypeptides or proteins encoded by the polynucleotides of the present disclosure or by degenerate variants of the polynucleotides of the present disclosure. By "degenerate variant" is intended polynucleotides which differ from a nucleic acid fragment of the present disclosure (e.g., an ORF) by nucleotide sequence but, due to the degeneracy of the genetic code, encode an identical polypeptide sequence. Preferred polynucleotides of the present disclosure are the ORFs that encode proteins or polypeptides. A variety of methodologies known in the art can be utilized to obtain any one of the isolated polypeptides or proteins of the present disclosure. At the simplest level, the amino acid sequence can be synthesized using commercially available peptide synthesizers. This is particularly useful in producing small peptides and fragments of larger polypeptides. Fragments are useful, for example, in generating antibodies against the native polypeptide. In an alternative method, the polypeptide or protein is purified from bacterial cells which naturally produce the polypeptide or protein. One skilled in the art can readily follow known methods for isolating polypeptides and proteins in order to obtain one of the isolated polypeptides or proteins of the present disclosure. These include, but are not limited to, immunochromatography, HPLC, size-exclusion chromatography, ion-exchange chromatography, and immuno-affinity chromatography. *See*, *e.g*., Scopes, Protein Purification: Principles and Practice, Springer-Verlag (1994); Sambrook, et al., in Molecular Cloning: A Laboratory Manual; Ausubel et al., Current Protocols in Molecular Biology*.* The polypeptides and proteins of the present disclosure can alternatively be purified from cells which have been altered to express the desired polypeptide or protein. As used herein, a cell is said to be altered to express a desired polypeptide or protein when the cell, through genetic manipulation, is made to produce a polypeptide or protein which it normally does not produce or which the cell normally produces at a lower level. One skilled in the art can readily adapt procedures for introducing and expressing either recombinant or synthetic sequences into eukaryotic or prokaryotic cells in order to generate a cell which produces one of the polypeptides or proteins of the present disclosure.

The protein or polypeptide of the disclosure may also be expressed as a product of non-human transgenic animals, e.g., as a component of the milk of transgenic cows, goats, pigs, or sheep which are characterized by somatic or germ cells containing a polynucleotide encoding the protein or polypeptide of the disclosure.

The proteins or polypeptides of the disclosure provided herein also include proteins or polypeptides characterized by amino acid sequences similar to those of purified proteins or polypeptides of the disclosure but into which modifications are naturally provided or deliberately engineered. For example, modifications in the peptide or DNA sequences can be made by those skilled in the art using known techniques. Modifications of interest in the protein sequences may include the alteration, substitution, replacement, insertion or deletion of a selected amino acid residue in the coding sequence. For example, one or more of the cysteine residues may be deleted or replaced with another amino acid to alter the conformation of the molecule. Techniques for such alteration, substitution, replacement, insertion or deletion are well known to those skilled in the art (see, e.g., U.S. Pat. No. 4,518,584). Preferably, such alteration, substitution, replacement, insertion or deletion retains the desired activity of the protein or polypeptide.

Other fragments and derivatives of the polypeptides or proteins of the disclosure which would be expected to retain protein activity in whole or in part and may thus be useful for screening or other immunological methodologies may also be easily made by those skilled in the art given the disclosures herein.

The protein or polypeptide of the disclosure may also be produced by operably linking a polynucleotide of the disclosure to suitable control sequences in one or more insect expression vectors, and employing an insect expression system. Materials and methods for baculovirus/insect cell expression systems are commercially available in kit form from, e.g., Invitrogen, San Diego, Calif., U.S.A. (the MaxBat.RTM. kit), and such methods are well known in the art, as described in Summers and Smith, Texas Agricultural Experiment Station Bulletin No. 1555 (1987). As used herein, an insect cell capable of expressing a polynucleotide of the present disclosure is "transformed."

The protein or polypeptide of the disclosure may be prepared by culturing transformed host cells under culture conditions suitable to express the recombinant protein or polypeptide. The resulting expressed protein or polypeptide may then be purified from such culture (i.e., from culture medium or cell extracts) using known purification processes, such as gel filtration and ion exchange chromatography. The purification of the protein or polypeptide may also include an affinity column containing agents which will bind to the protein or polypeptide; one or more column steps over such affinity resins as concanavalin A-agarose, heparin-toyopearl.RTM. or Cibacrom blue 3GA Sepharose.RTM.; one or more steps involving hydrophobic interaction chromatography using such resins as phenyl ether, butyl ether, or propyl ether; or immunoaffinity chromatography.

Alternatively, the protein or polypeptide of the disclosure may also be expressed in a form which will facilitate purification. For example, it may be expressed as a fusion protein, such as those of maltose binding protein (MBP), glutathione-S-transferase (GST) or thioredoxin (TRX). Kits for expression and purification of such fusion proteins are commercially available from New England BioLab (Beverly, Mass.), Pharmacia (Piscataway, N.J.) and In Vitrogen, respectively. The protein or polypeptide can also be tagged with an epitope and subsequently purified by using a specific antibody directed to such epitope. One such epitope ("Flag") is commercially available from Kodak (New Haven, Conn.).

Finally, one or more reverse-phase high performance liquid chromatography (RP-HPLC) steps employing hydrophobic RP-HPLC media, e.g., silica gel having pendant methyl or other aliphatic groups, can be employed to further purify the protein or polypeptide. Some or all of the foregoing purification steps, in various combinations, can also be employed to provide a substantially homogeneous isolated recombinant protein or polypeptide. The protein or polypeptide thus purified is substantially free of other mammalian proteins and is defined in accordance with the present disclosure as an "isolated protein."

As choice of antibody format, we preferred the Fab format above the scFv format, because the Fab format allows rapid high through-put affinity-screening assays for crude antibody preparations. Many scFv's indeed form higher molecular weight species including dimers (Weidner, et al., (1992) J. Biol. Chem. 267, 10281-10288; Holliger, et al., (1993) Proc. Natl. Acad. Sci. U. S. A. 90, 6444-6448) and trimers (Korttet al., (1997) Protein Eng. 10, 423-433), which complicate both selection and characterisation. We chose the Fab display format in which a variable domain from a heavy or light chain gene is linked to a phage coat protein, and in some embodiments, also carries a tag for detection and purification. The other chain is expressed as separate fragment secreted into the periplasm, where it can pair with the gene that is in a protein fusion with the phage coat protein (Hoogenboom, et al., (1991) Nucleic Acids Res. 19, 4133-4137). In some embodiments, the phage coat protein a pIII coat protein. In other embodiments, the variable domain from a heavy chain gene is fused to the phage coat protein and the light chain gene is expressed as a separate fragment.

The choice for the Fab format was based on the notion that the monomeric appearance of the Fab permits the rapid screening of large numbers of clones for kinetics of binding (off-rate) with crude protein fractions. This reduces the time for post-selection analysis dramatically when compared to that needed for selected single-chain Fv (scFv) antibodies from phagemid libraries (Vaughan, et al., (1996) Nat. Biotechnol. 14, 309-314; Sheets, et al., (1998) Proc. Natl. Acad. Sci. U. S. A. 95, 6157-6162), or Fab fragments from other phage libraries (Griffiths, et al., (1993) EMBO J. 12, 725-734).

The Fab library obtainable by the method of the invention produced on average 14 different Fab's against 6 antigens that were tested. These include tetanus toxoid, the hapten phenyl-oxazolone, the breast cancer associated MUC1 antigen and three highly related glycoprotein hormones: human Chorionic Gonadotropin ("hCG"), human Luteinizing Hormone ("hLH") and human Follicle Stimulating Hormone ("hFSH"). For the glycoprotein hormones, the Fab library obtainable by the method of the invention produced a panel of either homone-specific or cross-reactive antibodies. Thus, without using sophisticated selection protocols, hormone specific as well as cross-reactive Fab's were retrieved against these highly homologous glycohormones, demonstrating that the library is a rich source of antibody specificities. The affinities of the anti-glycohormone antibodies varied between 2.7 and 38 nM. Finally, the Fab-format indeed permitted the rapid screening and a reliable ranking of individual clones based on off-rate using crude fractions.

Furthermore, the specificities of the antibodies obtained by selections on the gonadotropins are unique: due to the high degree of homology between hLH and hCG it has been very difficult to isolate hCG specific monoclonal antibodies with the hybridoma technology, whereas there are very few hLH specific antibodies (Moyle, et al., (1990) *J. Biol.* Chem. **265**, 8511-8518; Cole, (1997) *Clin.* Chem. **43**, 2233-2243). Using a straight forward selection procedure, taking no precaution to avoid the selection of cross-reactive Fab's, we have readily isolated fragments with all possible specificities: Fab's specific for any of the three hormones hCG, hLH and hFSH, and cross-reactive Fab's recognizing the common α-chain or epitopes on the β-chain shared by hCG and hLH. These selections demonstrated that antibodies directed against different epitopes within single antigen molecules can be retrieved from the library. The Fab library obtainable by the method of the invention permits the monitoring of selections with polyclonal phage preparations and large scale screening of antibody off-rates with unpurified Fab fragments. Overall, antibodies with off-rates in the order of 10⁻² to 10⁻⁴ s⁻¹ and affinities up to 2.7 nM, were recovered. The kinetics of these phage antibodies are of the same order of magnitude as antibodies associated with a secondary immune response.

An indication that antibodies from the Fab library behave similarly or better than antibodies from a scFv library with regards to affinity comes from a comparison of selections of two different libraries on the same two antigens under identical conditions. Antibodies to MUC1 selected from a large naive scFv library (Henderikx et al., (1998) *Cancer Res.* **58**,4324-4332) have faster off-rates then the equivalent Fab's isolated from the library described in this study. Further, they show a very distinct V-gene usage and have a different fine specificity. Similarly, when comparing the off-rates of phage antibodies against the pancarcinoma marker Epithelial Glycoprotein-2, one of the Fab's selected from the present library appears to have a 10-fold slower off-rate than the best scFv (Vaughan et al., (1996) *Nat. Biotechnol.* **14**, 309-314).

The affinities of the selected antibody fragments are, however, very much dependent on the antigen used for selection. Sheets and colleagues reported an affinity varying between 26 and 71 nM for the selected scFv fragments specific for the anti-*Clostridia botulinum* neurotoxin type A fragments, whereas for antibodies to the extracellular domain of human ErbB-2, K_{d}'s between 0.22 and 4.03 nM were found (Sheets et al., (1998) Proc. Natl. Acad. Sci. U. S. A. 95, 6157-6162). The affinities of the gonadotropin specific Fab's selected from our library varied between 2.7 and 38 nM, which is comparable to the protein binding scFv's from the naive library made by Vaughan et al. and Sheets et al., and approaches the values of the best antibodies in their kind. The size of the Fab library obtainable by the method of the invention is not only important for affinity, but it also determines the success rate of selection of antibodies against a large set of different antigens. In this respect the Fab library obtainable by the method of the invention performs very well: over 24 antibodies to the hapten phOx, and on average 13 antibodies against the other antigens were selected.

In the limited set of 14 Fab clones that were sequenced, we identify antibodies with variable region genes from all large V-gene families, including V_{H1/3/4}, V_{κ1/3}, and V_{λ1/2}, but also less frequently used segments of family V_{H6}, V_{κ2/7} and V_{λ7} were retrieved. Most likely the use of an extended set of variable region gene primers, designed on the most recent sequence information of the germline V-regions, and/or the separate PCRs, combined with partially separate cloning, ensured access to a highly diverse sample of the human V-gene repertoire.

A library is prepared from polynucleotides which are capable of encoding the desired specific binding pair member. A variety of techniques exist for preparing the library, which may be prepared, for example, from either genomic DNA or cDNA. See, e.g., Sambrook et al., Molecular Cloning, A Laboratory Manual, 2nd ed., Cold Spring Harbor Laboratory Press, Cold Spring Harbor, N.Y., 1989. Cells may serve as the source of the polynucleotides which encode the specific binding pair members of interest. Enrichment procedures and means for amplifying the regions containing the gene(s), may be employed. For instance, when the desired specific binding pair member is an antibody, RNA and or genomic DNA may be prepared, for example, from spleen cells obtained from an unimmunized animal, from an animal immunized with target(s) of interest, from hybridoma cells, or from lymphoblastoid cells. The library of antibodies obtained from the unimmunized animals contain an unbiased representation of the entire antibody repertoire, while the library of antibodies obtained from the immunized animals contain a biased population of antibodies directed against epitopes of the target(s). Spleen cells, or immune cells from other tissues or the circulatory system may be obtained from a variety of animal species, such as human, mouse, rat, equine, bovine, avian, etc.

Amplification of messenger RNA (mRNA) isolated from cells of interest, such as spleen or hybridoma cells, may be performed according to protocols outlined in, e.g., U.S. Pat. No. 4,683,202, Orlandi, et al. Proc. Natl. Acad. Sci. USA 86:3833-3837 (1989), Sastry et al., Proc. Natl. Acad. Sci. USA 86:5728-5732 (1989), and Huse et al. Science 246:1275-1281 (1989), Abelson, J. and Simon, M. (eds), Methods in Enzymology, combinatorial chemistry, Vol. 267, San Diego: Academic Press (1996), Kay, B.K., Winter, J., McCafferty, J. (eds), Phage Display of peptides and Proteins, a Laboratory Manual, San Diego: Academic Press (1996). Oligonucleotide primers useful in amplification protocols may be unique or degenerate or incorporate inosine at degenerate positions. Thus, for multi-chain immunoglobulins, primers would be generally used for amplification of sequences encoding the variable regions of both the heavy and light chains. Restriction endonuclease recognition sequences may be incorporated into the primers to allow for the cloning of the amplified fragment into a vector in a predetermined reading frame for expression.

Expression libraries containing the amplified cDNA are typically prepared in a vector such as a bacteriophage or phagemid. The characteristics of the suitable bacteriophage or phagemid depends on the specific embodiment employed, and will generally be those which conveniently allow insertion of the recombinant polynucleotides into host cells by *in vitro* packaging or transformation.

Host cells are then infected with the phage or phagemid and helper phage, and cultivated under conditions allowing for the expression and assembly of phage particles. In one embodiment, the appropriate host cells for the bacteriophage or phagemids of the method of the invention are various strains of E. coli, specific examples depending on which of the several suitable vectors is chosen. Of course, phage or phagemid having bacterial hosts other than E. coli may also be used.

To enrich for and isolate phage particles or phage which contain cloned library sequences that encode a desired specific binding pair member, and thus to ultimately isolate the nucleic acid sequences themselves, phage particles or phage harvested from the host cells are affinity purified. A target or binding partner for the desired specific binding pair member is used in the affinity purification. For example, when the desired specific binding pair member is an antibody which specifically binds a particular target, the target is used to retrieve phage particles or phage having the desired antibody on its outer surface. The target is typically adsorbed to an insoluble substrate, such as a particle or bead or plate. The phage particles or phage so obtained may then be amplified by infecting into host cells (with helper phage for the phage particles containing the phagemids). Additional rounds of affinity enrichment and amplification may be employed until the desired level of enrichment is reached or the desired phage particles or phage are no longer enriched relative to the background phage particles or phage.

The enriched antibody-phage particles or phage are also screened with additional detection techniques such as expression plaque (or colony) lift (see, e.g., Young and Davis, Science, 222:778-782 (1983)) whereby the same or another binding partner is used as a probe. Screening may employ additional assays (for a catalytic activity, for example) which are used to detect, in situ, plaques expressing specific binding pair members having the desired characteristics. The phage particles or phage obtained from the screening protocol are infected into cells, propagated, and the phage particle or phage DNA isolated and sequenced, and/or recloned into a vector intended for gene expression in prokaryotes or eukaryotes to obtain larger amounts of the selected specific binding pair member.

In another embodiment, the specific binding pair member encoded in the library (or multiple chains comprising said specific binding pair member) is transported to an extra-cytoplasmic compartment of the host cell, usually the periplasmic space, to facilitate processing and/or proper assembly. When extra-cytoplasmic transport of the desired specific binding pair member is employed, the sequences encoding the specific binding pair member are cloned adjacent to appropriate transcriptional and translational signals and signal peptide leaders that will direct the mature chains to the periplasm. As above, at least one of the chains is cloned as a fusion protein with a phage coat protein so that the phage coat protein does not substantially interfere with the ability of the specific binding pair member of interest to bind a target which is used in the affinity enrichment protocol.

A preferred example of this embodiment is the placement of a specific binding pair member in the N-terminus region of the minor coat protein pIII of bacteriophage fd. Before incorporation into the phage, pIII resides in the inner membrane of the host cell with its N-terminus protruding into the periplasm. In this configuration the polypeptide of a specific binding pair member in the N-terminus of pIII is available for binding to other polypeptide chains that make up the specific binding pair member of interest. This complex is then incorporated into the mature phage particle or phage as it exits the cell and the C-terminus embeds in the coat of the phage particle or phage.

In this embodiment the synthesis and amplification of polynucleotides is as described above, and then is cloned into or near a vector sequence encoding a coat protein, where the vector is, or is derived from, a filamentous phage, such as f1, fd, Pf1, M13, etc. In a preferred embodiment the filamentous phage is fd-tet. The phage vector is chosen to contain a cloning site located in the 5' region of a gene encoding a phage coat protein, such as, for example, the pIII coat protein. An appropriate vector (e.g., fd-tet B1 which is described below) allows oriented cloning of foreign sequences so that they are expressed at or near the N-terminus of the mature coat protein. A library is constructed by cloning the polynucleotides (e.g., the V_{H} region) from the donor cells into a coat protein gene (e.g., gene III, "gIII") cloning site. The cloned sequences of, for example, the V_{H} domains are ultimately expressed as polypeptides or proteins fused to the N-terminus of the mature coat protein on the outer, accessible surface of the assembled phage particles or phage.

When the desired protein is a multi-chain protein, such as an antibody or binding fragment thereof, the polynucleotide encoding the chain(s) not cloned into a phage coat protein may be cloned directly into an appropriate site (as described below) of the vector containing the first chain-coat protein library; or, preferably, the subsequent chain(s) may be cloned as a separate library in a different plasmid vector, amplified, and subsequently the fragments installed in the first chain-coat protein library vector. For example, when the first chain is an antibody heavy chain or binding fragment thereof, the ultimate destination of light chain V_{L} cDNA sequence is in a vector that already contains a V_{H} sequence in a coat protein gene, thus randomly recombining V_{H} and V_{L} sequences in a single vector.

The second or subsequent chain of the desired multi-chain protein, such as V_{L}, is cloned so that it is expressed with a signal peptide leader sequence that will direct its secretion into the periplasm of the host cell. For example, several leader sequences have been shown to direct the secretion of antibody sequences in E. coli, such as OmpA (Hsiung, et al., Biotechnology 4:991-995 (1986)), pelB (Better, et al., Science 240:1041-1043 (1988)), phoA (Skerra and Pluckthun, Science 240:1038-1043 (1988)), beta-lactamase (Zemel-Dreasen and Zamir, Gene 27:315-322 (1984)), and those described in Abelson, J. and Simon, M. (eds), Methods in Enzymology, combinatorial chemistry, Vol. 267, San Diego: Academic Press (1996), and Kay, B.K., Winter, J., McCafferty, J. (eds), Phage Display of peptides and Proteins, a Laboratory Manual, San Diego: Academic Press (1996).

Generally, the successful cloning strategy utilizing a phage coat protein, such as pIII of filamentous phage fd, will provide: (1) expression of a protein chain (or a first polypeptide chain when the desired protein is multichained, e.g., the V_{H} chain) fused to the N-terminus of a full sized (or nearly full sized) coat protein (e.g., pIII) and transport to the inner membrane of the host where the hydrophobic domain in the C-terminal region of the coat protein anchors the fusion protein in the membrane, with the N-terminus containing the chain protruding into the periplasmic space and available for interaction with a second or subsequent chain (e.g., V_{L} to form an Fab fragment) which is thus attached to the coat protein; and (2) adequate expression of a second or subsequent polypeptide chain if present (e.g., V_{L}) and transport of this chain to the soluble compartment of the periplasm.

In one embodiment for affinity enrichment of desired clones, about 10³ to 10⁴ library equivalents (a library equivalent is one of each recombinant--10⁴ equivalents of a library of 10⁹ members is 10⁹ x 10⁴ = 10¹³ phage particles or phage) are incubated with target to which the desired specific binding pair member (e.g., antibody) is sought. The target is in one of several forms appropriate for affinity enrichment schemes. In one example the target is immobilized on a surface or particle, optionally anchored by a tether of enough length (3 to 12 carbons, for example) to hold the target far enough away from the surface to permit free interaction with the antibody combining site. The library of phage particle or phage bearing antibodies is then panned on the immobilized target generally according to procedures well-known in the art, for example, those described in Abelson, J. and Simon, M. (eds), Methods in Enzymology, combinatorial chemistry, vol. 267, San Diego: Academic Press (1996), Kay, B.K., Winter, J., McCafferty, J. (eds), Phage Display of peptides and Proteins, a Laboratory Manual, San Diego: Academic Press (1996).

A second example of target presentation is target attached to a recognizable ligand (again optionally with a tether of some length). A specific example of such a ligand is biotin. The target, so modified, is incubated with the library of phage particles or phage and binding occurs with both reactants in solution. The resulting complexes are then bound to streptavidin (or avidin) through the biotin moiety. The streptavidin may be immobilized on a surface such as a plastic plate or on particles, in which case the complexes are physically retained; or the streptavidin may be labelled, with a fluorophore, for example, to tag the active phage/antibody for detection and/or isolation by sorting procedures, e.g., on a fluorescence-activated cell sorter.

In one embodiment, the phage particles or phage bearing antibodies without the desired specificity are removed by various means, for example, by washing. The degree and stringency of washing required will be determined for each specific binding pair member of interest. A certain degree of control can be exerted over the binding characteristics of the antibodies recovered by adjusting the conditions of the binding incubation and the subsequent washing. The temperature, pH, ionic strength, divalent cations concentration, and the volume and duration of the washing will select for antibodies within particular ranges of affinity for the hapten. Selection based on slow dissociation rate, which is usually predictive of high affinity, is the most practical route. This may be done either by continued incubation in the presence of a saturating amount of free hapten, or by increasing the volume, number, and length of the washes. In each case, the rebinding of dissociated antibody-phage is prevented, and with increasing time, antibody-phage of higher and higher affinity are recovered.

Antibodies with certain catalytic activities may be enriched in groups of antibodies with high affinity for reactants (substrates and intermediates) but low affinity for products. A double screen to enrich for antibodies with these characteristics may be useful in finding antibodies to catalyze certain reactions. Further, catalytic antibodies capable of certain cleavage reactions may also be selected. One category of such reactions is the cleavage of a specific end group from a molecule. For example, a catalytic antibody to cleave a specific amino acid from an end of a peptide may be selected by immobilizing the peptide and panning the antibody library under conditions expected to promote binding but not cleavage (e.g., low temperature, particular ionic strength, pH, cation concentration, etc., depending on the nature of the end group and the cleavage reaction) and followed by a wash. This allows antibodies that recognize the end group to bind and become immobilized, and from this group will come those capable of cleavage. To find those capable of cleavage, the conditions are shifted to those favorable for cleavage. This step will release those antibody-phage capable of cleaving themselves free of the immobilized peptide.

An alternative way to accomplish this is to pan for antibodies that bind to the specific end group by attaching that end group to a bond different from that to be cleaved (a non-peptide bond, for example). By subsequent panning (of the positive phage from the first screen) on the end group attached via the proper bond under cleavage conditions, the non-binding fraction will be enriched for those with the desired catalytic activity.

To elute the active antibody-phage particle or phage from the immobilized target, after washing at the appropriate stringency, the bound (active) phage particle or phage can be recovered by eluting with pH shift. For example, pH2 or pH11 may be used, which is then neutralized and the eluted phage are amplified by infecting or transforming the host cells. The cells are then grown as tetracycline resistant colonies. The colonies are scraped up and the extruded phage are purified by standard procedures as before. These phage are then used in another round of affinity enrichment (panning), and this cycle is repeated until the desired level of enrichment is reached or until the target phage are no longer enriched relative to the background phage particles or phage. To isolate individual clones, phage particles or phage from the final round of panning and elution are infected into cells or their DNA is transformed into cells and grown on agar (usually L-agar) and antibiotics (usually tet) to form well separated individual colonies, each of which is a clone carrying vectors with both V_{H} and V_{L} sequences. The single stranded DNA from phage particles or phage extruded from each colony may be isolated and DNA coding for the V_{H} and V_{L} fragments sequenced. The replicative form of the phage DNA (double stranded) may be isolated by standard means and the DNA in the cloning sites (V_{H} and V_{L} sequences) recloned into a vector designed for gene product expression in prokaryotes or eukaryotes to obtain larger amounts of the particular antibodies selected in the screening process.

Phage identified as having an antibody recognized by the target ligand are propagated as appropriate for the particular phage vector used. For fd-tet this is done in a liquid culture of rich medium (L-broth, for example) with antibiotic (Tet) selection. The phage are harvested and DNA prepared and sequenced by standard methods to determine the DNA and amino acid sequence of the particular antibody.

The DNA may be recloned in a suitable eukaryotic or prokaryotic expression vector and transfected into an appropriate host for production of large amounts of protein. Antibody is purified from the expression system using standard procedures. The binding affinity of the antibody is confirmed by well known immunoassays with the target antigen or catalytic activity as described in Harlow and Lane, Antibodies, A Laboratory Manual, Cold Spring Harbor, N.Y. (1988), Abelson, J. and Simon, M. (eds), Methods in Enzymology, combinatorial chemistry, vol. 267, San Diego: Academic Press (1996), Kay, B.K., Winter, J., McCafferty, J. (eds), Phage Display of peptides and Proteins, a Laboratory Manual, San Diego: Academic Press (1996).

In another embodiment, phage particles or phage displaying the desired specific binding pair member are affinity purified as follows: approximately 10³ -10⁴ library equivalents of phage particles or phage are reacted overnight with 1 micropgram purified antibody at 4°C. The mixture is panned by a procedure as follows. A polystyrene petri plate is coated with 1 ml of streptavidin solution (1 mg/ml in 0.1 M NaHCO₃, pH 8.6, 0.02% NaN₃) and is incubated overnight at 4°C. The following day the streptavidin solution is removed. The plate is filled with 10 ml blocking solution (30 mg/ml BSA, 3 micrograms/ml streptavidin in 0.1 M NaHCO₃, pH 9.2, 0.02% NaN₃) and incubated for 2 hours at room temperature. Two micrograms of biotinylated goat anti-mouse IgG (BRL) are added to the antibody-reacted library and incubated for 2 hours at 4°C. Immediately before panning, blocking solution is removed from streptavidin coated plate, and the plate is washed 3 times with TBS/0.05% Tween 20. The antibody-reacted library is then added to the plate and incubated for 30 minutes at room temperature. Streptavidin coated agarose beads (BRL) may also be used for this affinity purification. The library solution is removed and the plate is washed ten times with TBS/0.05% Tween 20 over a period of 60 minutes. Bound phage are removed by adding elution buffer (1 mg/ml BSA, 0.1N HCl, pH adjusted to 2.2 with glycine) to the petri plate and incubating for 10 minutes to dissociate the immune complexes. The eluate is removed, neutralized with 2M Tris (pH unadjusted) and used to infect log phase F'-containing bacterial cells. T hese cells are then plated on LB agar plates containing tetracycline (20 .mu.g/ml), and grown overnight at 37°C. Phage -particles or phage are isolated from these plates as described and the affinity purification process was repeated for two to three rounds. After the final round of purification, a portion of the eluate is used to infect cells and plated at low density on LB tetracycline plates. Individual colonies are transferred to culture tubes containing 2 ml LB tetracycline and grown to saturation. Phage or phagemid DNA is isolated using a method designed for the Beckman Biomek Workstation (Mardis and Roe., Biotechniques, 7:840-850 (1989)) which employs 96-well microtiter plates. Single stranded DNA is sequenced by the dideoxy method using Sequenase (U.S. Biochemicals) and an oligonucleotide sequencing primer (5'-CGATCTAAAGTTTTGTCGTCT-3' SEQ ID NO: 2) which is complementary to the sequence located 40 nucleotides 3' of the second BstXI site in fdTetB1.

We considered a number of variables to address in the construction of a novel, very large antibody phage library: (i) the primer design was optimised for amplification of variable gene pools to maintain maximum diversity; (ii) a highly efficient two-step cloning method was developed to obtain a very large naive library; (iii) an antibody format and compatible cloning vector were chosen, which should permit the rapid down-stream analysis of selected clones. In order to achieve access to as many different human heavy and light chain V-region gene segments as possible, a new set of oligonucleotide primers was developed (Table I), the design of which was based on the most recent sequence information provided by the V-base.

The primers were designed to be the or several consensus sequences which would have at least a 70% homology to the respective 5' or 3' end based coding region in the human germ line gene segments of the specific V gene family they would have to amplify. The primers would amplify at least one V-gene segment using the PCR conditions described below, and in one embodiment are appended with appropriate positioned restriction sites for cloning into the vector for Fab expression.

The primers should allow efficient amplification of all commonly used V-gene segments. Further, to obtain the large sized Fab libraries in accordance with the invention (over 10¹⁰ in diversity), we used a two-step cloning procedure: heavy and light chain variable genes were first separately cloned as digested PCR products, and were then combined by restriction fragment cloning to form a large library of Fab fragments. This cloning procedure should be a more efficient route for library construction than the relatively inefficient direct cloning of digested PCR-products, while avoiding the DNA instability often associated with *in vivo* recombination systems (Griffiths, et al., (1994) EMBO J. 13, 3245-3260).

A new phagemid vector, pCES1 (Fig. 1), was constructed, that allows the stepwise cloning of antibody fragments in Fab format. In this vector system, the variable heavy chain region genes are cloned as V_{H}-gene fragments; the vector supplies all Fab's with a human gamma-1 C_{H1} domain. The V_{H}C_{H1} formed by insertion of the V_{H}-gene fragments to the vector is fused (in the vector) to two tags for purification and detection (a histidine tail for Immobilised Metal Affinity Chromatography (Hochuli, et al., (1988) BioTechnology 6, 1321-1325) and a c-*myc*-derived tag (Munro, et al., H. R. (1986) Cell 46, 291-300)), followed by an amber stop codon (Hoogenboom, et al., (1991) Nucleic Acids Res. 19, 4133-4137) and the minor coat protein III of filamentous phage f_{d}. The antibody light chain is cloned as full V_{L}C_{L} fragment, for directed secretion and assembly with the V_{H}C_{H1} on the phage particle.

In one embodiment, the vector comprises an expression cassette with a bicistronic or double cistronic expression cassette to allow linked (for the bicistronic) or independent (for the double cistronic) expression of the antibody light and heavy chain or their fusions, such expression cassette consisting of the following elements : (1) a promoter suited for non-inducible and inducible expression (e.g lacZ); (2) a ribosome binding site and signal sequence preceeding the light and heavy chain cloning regions; (3) possible, but not necessarily, a region following the heavy or light chain cloning region that encodes a tag sequence such as a stretch of 5-6 histidines or a sequence recognised by an antibody and an amber codon; (4) a phage coat protein encoded as a fusion to the 3' end of either the heavy or light chain.

This new phage library will be a valuable source of antibodies to essentially any target. The antibodies may be used as research reagents or as starting point for the development of therapeutic antibodies or agricultural products. As the list of sequenced genomes and disease-related gene products is expanding rapidly, there will be a growing need for an *in vitro* and eventually automated method for antibody isolation. As antibodies have been and will be ideal probes for investigating the nature, localization and purification of novel gene products, this library is envisaged to play an important role in target validation and target discovery in the area of functional genomics.

Protein variants expressed on the surface of bacteriophage have been selected on the basis of their affinity for ligand (antigen) using chromatography, panning or adsorption to cells. Elution from affinity matrices has been achieved by specific elution using the ligand (antigen or a related compound) or non-specific elution using, for example, 100mM triethylamine. Washing procedures remove non-specifically bound phage. The phage binds to and is eluted from the matrix according to the affinity or the nature of the binding interaction. Specifically eluted phage are then used to infect male *E. coli* cells expressing the F pilus, allowing recovery of phage containing DNA encoding proteins with the desired binding characteristics.

Selection can be made not only on the basis of specificity, but also on the basis of affinity. Separation is readily attainable by affinity chromatography between phage expressing an antibody with a dissociation constant of 10⁻⁸ M and one with a dissociation constant of 10⁻⁵ M. Clackson, T. et al. (1991). Nature 352: 624-628. The isolation of the latter antibody from an immune repertoire demonstrates that antibodies with affinities characteristic of the primary immune response can be isolated using phage technology.

Antibodies directed against cell surface antigens can also be isolated by selective adsorption of phage on the surface of cells. Similarly, it may be possible to incorporate negative selection with cells to remove undesired cross-reactivities with cell surface markers. As these are rather difficult and as yet poorly understood methods, methods based on the selection on purified antigen should be used whenever possible.

Any selection for binders within a population will automatically tend to select for high affinity variants at the expense of the lower, enriching the high affinity population. This has been used to good effect recently in the isolation of high affinity human antibodies from a naive repertoire. Marks, J.D. et al. (1991) J. Mol. Biol. 222, 581-597. For optimal selection, the antigen concentration should be less than the affinity constant. This should be borne in mind when isolating an antibody with pre-defined characteristics. Further details on various selection methods is given in the reviews in this manual.

With such large panels of antibodies isolated, it is useful to have methods available to readily determine the kinetic parameters of each individual antibody-antigen interaction. We have shown that it is feasible to rapidly and accurately determine the off-rate of non-purified antibodies in periplasmic fractions prepared from small scale cultures using surface plasmon resonance. Using this method, a series of tetanus toxoid specific Fab's showed a monophasic dissociation, which is expected for a truly monomeric Fab-fragment binding to a low density antigen surface. Using this off-rate screening assay, we determined the off-rates for the best tetanus toxoid and MUC1 specific Fab's to be in the order of 10⁻² to 10⁻⁴ s⁻¹.

We tested the integrity of selected Fab's obtained from periplasmic fractions using western blots. When incubated in non-reducing sample buffer, two products were detected with the 9E10 antibody, which recognises the *myc*-tag at the end of the CH1 domain, the major product is the intact Fab-molecule, in which an intermolecular disulfide bridge covalently links heavy and light chain fragments; the low molecular product is most likely derived from non disulfide bridge linked heavy chains. Analysis with anti-light chain sera reveals a similar pattern and shows that the clones use a nearly equal percentage of kappa and lambda chains (found in six and seven clones respectively of a total of 13 tested). Upon reduction of purified, functional antigen-binding Fabs, equal amounts of heavy and light chain are seen, while under non-reducing conditions, the main product is represented by the disulphide linked Fab-molecule, with an equal amount of the non-covalently linked V_{H}C_{H1} and V_{L}C_{L} products visible. Production yields of selected hormone specific Fab's varied between 160 µg and 1.43 mg Fab per litre culture, which was in the same range as was found for the unselected Fabs.

A panel of 14 antigen-specific Fab's was fully sequenced (3 anti-MUC1 antibodies; 11 anti-gonadotropin antibodies). The heavy chain genes are derived from the four largest V_{H} families (V_{H1}, V_{H3}, V_{H4} and V_{H6}); the V_{L} genes belong to one of four V_{κ}-families or one of three V_{λ}-families. Chain promiscuity is seen for the α-chain specific clone SC#4G, the α/β-LH specific clones LH#2H and LH#3G, and β-FSH specific clone FS#8B, which all used a highly homologous V_{κ2} light chain gene segment combined with different heavy chain fragments. The 3 anti-MUC1 antibodies use heavy and light chain genes derived from 2 different VH and V6 families; clone MUC#9 uses a VH with a cross-over of 2 segments.

The present invention is further illustrated in the following examples.

As source of lymphoid tissues we used peripheral blood lymphocytes from 4 healthy donors and part of a tumor-free spleen removed from a patient with gastric carcinoma. B lymphocytes were isolated from 2-L of blood on a Ficoll-Pacque gradient. For RNA isolation, the cell pellet was immediately dissolved in 50 ml 8 M guanidinium thiocyanate / 0.1 M 2-mercaptoethanol (Chirgwin, et al., (1979) Biochemistry 18, 5294-5299). Chromosomal DNA was sheared to completion by passing through a narrow syringe (1.2/0.5 mm gauge), and insoluble debris was removed by low speed centrifugation (15 min 2,934xg at room temperature). RNA was pelleted by centrifugation through a CsCl-block gradient (12 ml supernatant on a layer of 3.5 ml 5.7 M CsCl / 0.1 M EDTA; in total 4 tubes) during 20 h at 125,000xg at 20°C in a SW41-rotor (Beckman). The yield of total RNA was approx. 600 µg. RNA was stored at -20^{E}C in ethanol. From the spleen, 2 g of tissue was used for homogenisation with a polytron in 20 ml 8 M guanidinium thiocyanate / 0.1 M 2-mercaptoethanol. The total volume was increased to 80 ml with guanidinium thiocyanate buffer, and after passage through a narrow syringe for shearing and removal of debris, RNA was pelleted as described before, except for 15 h at 85,000xg at 20°C in a SW28.1 rotor (12 ml supernatant on 3.5 ml 5.7 M CsCl / 0.1 M EDTA in 5 SW28.1 tubes). From 2 g of tissue, 3 mg of total RNA was extracted.

Random primed cDNA was prepared with 250 µg PBL RNA, while in a separate reaction 300 µg spleen RNA was used as template. RNA was heat denatured for 5 min at 65°C in the presence of 20 µg random primer (Promega), subsequently buffer and DTT were added according to the suppliers instructions (Gibco-BRL), as well as 250 µM dNTP (Pharmacia), 800 U RNAsin (40 U/µl; Promega) and 2,000 U MMLV-RT (200 U/µl; Gibco-BRL) in a total volume of 500 µl. After 2 h at 42°C, the incubation was stopped by a phenol / chloroform extraction; cDNA was precipitated and dissolved in 85 µl water.

Oligonucleotides used for PCR amplification of human heavy and light chain V-regions are described in Figure 2. IgM-derived heavy chain variable regions were obtained by a primary PCR with an IgM constant region primer. All primary PCRs were carried out with separate BACK primers and combined FOR primers, to maintain maximal diversity. The PCR-products were reamplified with a combination of JHFOR-primers, annealing to the 3' end of V_{H}, and Sfi-tagged VHBACK-primers, annealing to the 5' end, and subsequently cloned as V_{H}-fragments. The light chain V-genes of the kappa and lambda families were obtained by PCR with a set of CKFOR- or CλFOR-primer annealing to the 3' end of the constant domain and BACK-primers, priming at the 5' end of the V-regions. The DNA-segments were reamplified with primers tagged with restriction sites and cloned as V_{κ}C_{κ}- and V_{λ}C_{λ}-fragments.

PCR was performed in a volume of 50 µl using AmpliTaq polymerase (Cetus) and 500 pM of each primer for 28 cycles (1 min at 94°C, 1 min at 55°C and 2 min at 72°C), 9 separate IgM derived VH-amplifications were generated with 2 µl random primed cDNA (equivalent to 6 µg PBL RNA or to 7 µg spleen RNA) as template for each reaction. For the light chain families, 6 different V_{κ}C_{κ} products and 11 V_{λ}C_{λ}-products (C_{λ2}- and C_{λ7}-primers combined in each reaction) were obtained. All products were purified from agarose gel with the QIAex-II extraction kit (Qiagen). As input for reamplification to introduce restriction sites, 100-200 ng purified DNA-fragment was used as template in a 100 µl reaction volume. The large amount of input, ensuring the maintenance of variability, was checked by analysis of 4 λI of the "unamplified" PCR-mixture on agarose gel.

For the construction of the primary heavy chain and the two primary light chain repertoires, the PCR-products, appended with restriction sites, were gel purified prior to digestion and the different V_{H}-, V_{κ}- and V_{λ}-families combined into three groups. The V_{κ}C_{κ}- and V_{λ}C_{λ}-fragments were digested with ApaLI and AscI, and cloned into the phagemid vector pCES1. The V_{H}-fragments, 1.5 µg in total, were digested with *Sfi*I and *Bst*EII and ligated in a 100-200 µl reaction mixture with 9 U T₄-DNA ligase at room temperature to 4 µg, gel-purified vector pUC119-CES1 (similar to vector pCES1, but with the pIII gene deleted). The desalted ligation mixture for light or heavy chain pools was used for electroporation of the *E. coli* strain TG1, to create the one-chain libraries.

The Fab library was obtained by cloning of V_{H} fragments, digested from plasmid-DNA prepared from the heavy chain repertoires, into the plasmid collection containing the light chain repertoires. Plasmid DNA isolated from at least 3x10⁹ bacteria of the V_{H} library was digested with *Sfi*I and *Bst*EII for cloning in the vector that already container λ and κ light chain libraries. To retain clones with internal *Bst*EII site in the Vλ_ (this site is relatively frequent in some A germline V-segments (Persic, et al., (1997) Gene 187, 9-18), and also in the constant domain of one of the λ families), the cloning of V_{H}C_{H1} in the λ light chain repertoire containing vector was also carried out using *Sfi*I and *Not*I cloning sites, to create a less restriction-biased V_{λ_}libary.

The rescue of phagemid particles with helper phage M13-KO7 was performed according to (Marks, et al., (1991) J. Mol. Biol. 222, 581-597) on 10-L scale, using representative numbers of bacteria from the library for inoculation, to ensure the presence of at least 10 bacteria from each clone in the start inoculum. For selections, 10¹³ cfu's (colony forming units) were used with antigens immobilised in immunotubes (Maxisorp tubes, Nunc) (Marks, et al., (1991) J. Mol. Biol. 222, 581-597) or with soluble biotinylated antigens (Hawkins, et al., (1992b) J. Mol. Biol. 226, 889-896). The amount of the immobilised antigens tetanus toxoid and the hapten phenyl-oxazolone (conjugated to BSA in a ratio of 17 to 1) was reduced 10-fold during subsequent selection rounds, starting at 100 µg/ml at round 1. Capture with biotinylated antigen in solution was used for a 100-mer peptide encoding five copies of the tandem repeat of MUC1 (Henderikx, et al., (1998) Cancer Res. 58,4324-4332), or with human Chorionic Gonadotropin (hCG), human Luteinizing Hormone (hLH), human Follicle Stimulating Hormone (hFSH) and its chimeric derivative (hFSH-CTP, containing the carboxy terminal peptide from the hCG β-subunit fused to the β-subunit of hFSH). Antigens were biotinylated at a ratio of ten to twenty molecules NHS-Biotin (Pierce) per molecule antigen according to the suppliers recommendations. Unless stated otherwise, the antigens were used for selection at concentrations of 100 nM, 30 nM and 10 nM during round 1, 2 and 3 respectively. For hFSH-CTP 50, 15 and 10 nM was used respectively; for MUC1 peptide, 500, 100, 20 and 5 nM was used.

Soluble Fab was produced from individual clones as described before (Marks, et al., (1991) J. Mol. Biol. 222, 581-597). Culture supernatants were tested in ELISA with directly coated antigen or indirectly captured biotinylated antigen via immobilised biotinylated BSA-streptavidin. Tetanus toxoid and phOx-BSA were coated at 10 µg/ml in 0.1 M NaHCO₃ pH 9.6 for 16 h at 4°C. For coating of hCG and hFSH-CTP a concentration of 4 µg/ml in 50 mM NaHCO₃ pH 9.6 was used. For capture of biotinylated antigens, biotinylated BSA was coated at 2 µg/ml in PBS during 1 h at 37°C. After 3 washes with PBS-0.1 % (v/v) Tween 20 (PBST), plates were incubated during 1 h with streptavidin (10 µg/ml in PBS / 0.5% gelatin) (Henderikx, et al., (1998) Cancer Res. 58, 4324-4332). Following washing as above, biotinylated antigen was added for an overnight incubation at 4°C at a concentration of 0.5 µg/ml for MUC-1 peptide, 3 µg/ml for hLH, and 0.6 µg/ml for hFSH (binding to hCG was tested with directly coated antigen). The plates were blocked during 30 min at room temperature with 2% (w/v) semi-skimmed milk powder (Marvel) in PBS. The culture supernatant was diluted 1 or 5-fold in 2% (w/v) Marvel / PBS and incubated 2 h; bound Fab was detected with anti-myc antibody 9E10 (5 µg/ml) recognising the *myc*-peptide tag at the carboxyterminus of the heavy Fd chain, and rabbit anti-mouse-HRP conjugate (DAKO) (Marks, et al., (1991) J. Mol. Biol. 222, 581-597). Following the last incubation, staining was performed with tetramethylbenzidine (TMB) and H₂O₂ as substrate and stopped by adding half a volume of 2 N H₂SO₄; the optical density was measured at 450 nm. Clones giving a positive signal in ELISA (over 2x the background), were analysed by *Bst*NI-fingerprinting of the PCR-products obtained by amplification with the oligonucleotide primers M13-reverse and genelll-forward (Marks, et al., (1991) J. Mol. Biol. 222, 581-597).

Large-scale induction of soluble Fab fragments from individual clones was performed on 50 ml scale in 2xTY containing 100 µg/ml ampicillin and 2% glucose. After growth at 37°C to an OD₆₀₀ of 0.9, the cells were pelleted (10 min at 2,934xg) and resuspended in 2xTY with ampicillin and 1 mM IPTG. Bacteria were harvested after 3.5 h growing at 30°C by centrifugation (as before); periplasmic fractions were prepared by resuspending the cell pellet in 1 ml ice cold PBS. After 2 to 16 h rotating head-over-head at 4°C, the spheroplasts were removed by two centrifugation steps: after spinning during 10 min at 3,400xg, the supernatant was clarified by an additional centrifugation step during 10 min at 13,000xg in an eppendorf centrifuge. The periplasmic fraction obtained was directly used for determination of fine specificities by surface plasmon resonance or for western blot studies.

For sequencing, plasmid DNA was prepared from 50 ml cultures grown at 30°C in LB-medium, containing 100 µg/ml ampicillin and 2% glucose, using the QIAGEN midi-kit (Qiagen). Sequencing was performed with the thermocycling kit (Amersham) with CY5-labeled primers CH1 FOR (5'-GTC CTT GAC CAG GCA GCC CAG GGC-3' - SEQ ID NO: 3) and M13REV (5'-CAG GAA ACA GCT ATG AC-3' - SEQ ID NO: 4); samples were run on the ALF-Express (Pharmacia). V-gene sequences were aligned to V-base (Tomlinson et al., V-BASE, MRC Centre for Protein Engineering, 1997, http://www.mrc-cpe.cam.ac.uk/imt-doc/public/INTRO.html) or the Sanger Centre (Sanger Centre Germline Query, 1997, http//www.sanger.ac.uk/Data Search/gq-search.html).

An hCG-preparation purified from urine and immuno-affinity purified recombinant hLH, hFSH and hFSH-CTP produced in CHO-cells (Matzuk, et al., (1989) J. Cell. Biol. 109, 1429-1438; Muyan, et al., (1996) Mol. Endocrinol. 10, 1678-1687) were used for western blot studies as was described (Moyle, et al., (1990) J. Biol. Chem. 265, 8511-8518). Between 0.5 and 1 µg of each hormone was loaded per lane; proteins were diluted in non-reducing sample buffer and boiled during 5 min or directly applied on gel without heat-treatment; proteins were transferred to blotting membrane by electrotransfer. Blots were subsequently incubated for 16 h at room temperature with a 10-fold diluted periplasmic fraction in PBS / 4% Marvel. Bound Fab was detected with anti-*myc* antibody 9E10 (5 µg/ml) and 4,000-fold diluted anti-mouse alkaline phosphatase-conjugate (Promega), using the substrates 5-bromo-1-chloro-3-indolyl phosphate (BCIP) and nitro blue tetrazolium (NBT) (Boehringer Mannheim) for visualisation.

The specificity of the Fab's was further characterised by surface plasmon resonance (BIAcore 2000, Biacore). Recombinant hLH, hFSH and the urinary hCG were immobilised on the flow-cells of a CM-chip using the NHS/EDC-kit (Pharmacia), yielding a surface of 1906 RU for hLH, 1529 RU for hFSH and 1375 RU for hCG. Periplasmic fractions were diluted three-fold in Hepes Buffered Saline (HBS; 10 mM Hepes, 3.4 mM EDTA, 150 mM NaCl, 0.05% (v/v) surfactant P20, pH 7.4) and analysed using a flow rate of 10 µl/min.

Fab's were obtained by refolding of the total bacterial proteins from a 50 ml culture (de Haard, et al., (1998) Protein Eng., 11:1267-1276). Briefly, the pelleted cells from a 50 ml induced bacterial culture were resuspensed in 8 ml 8 M urea (in PBS). After sonication, the mixture was rotated head over head for 30 min and insoluble material was removed by centrifugation for 30 min at 13,000xg. The supernatant was dialysed against PBS with four buffer changes. Insoluble proteins were removed by centrifugation and the flow through fraction, obtained by filtration through a 0.2 µm membrane, was immediately loaded on an hCG column (bed volume 0.3 ml). The column material was prepared by coupling 8.4 mg protein to one gram Tresyl sepharose according to the suppliers instructions (Pierce). The column (1 ml column material) was washed with 10 volumes 100 mM Tris, 500 mM NaCl pH 7.5, subsequently with 10 volumes 100 mM Tris / 500 mM NaCl pH 9.5 and with 2 volumes 0.9% NaCl, bound Fab was eluted with two volumes 0.1 M TEA and immediately neutralised with 0.5 volume 1 M Tris pH 7.5. The Fab fraction was dialysed against PBS using a Microcon 30 spin dialysis filter (Amicon). Finally, a gel-filtration analysis was carried out on a Superdex 75HR column (Pharmacia). The yield was determined by measuring the optical density at 280 nm (using a molar extinction coefficient of 13 for Fab's). The kinetics of binding were analysed by surface plasmon resonance on three different hCG surfaces (303 RU, 615 RU and 767 RU immobilised, with 4955 RU BSA on a separate flow cell as a negative control). Fab present in crude periplasmic extracts was quantified on a high density surface of purified anti-human-Fab polyclonal antibody (Pierce) as described (Kazemier, et al., (1996) J. Immunol. Methods 194, 201-209). Anti-hCG Fab's controls were purified by affinity chromatography on hCG columns as described above and used to calibrate the system. The Fab library was constructed in two-steps. In the first step, variable region gene pools were amplified from approx. 4x10⁸ B-cells from the PBLs of four healthy donors, and, as a source of possibly more heavily mutated IgM antibodies, from a segment of a (tumor-free) spleen removed from a patient with gastric carcinoma, containing approximately 1.5x10⁸ B-cells (Roit, et al., (1985) Immunology, Gower Medical Publishing, Ltd., London). Only IgM-derived V_{H} segments were amplified by using an amplification with an oligonucleotide primer located in the first constant domain of this isotype. These products were cloned into phagemid vector pCES1 for V_{L}, and in pUC119-CES1 for V_{H} (cloning was more efficiently in the smaller sized vector, in which gene III was deleted). The PBL and spleen derived V_{H}, V_{κ} and V_{λ}-libraries were cloned separately to maintain diversity, to yield one-chain libraries in size typical for libraries made by cloning of PCR-fragments (Marks, et al., (1991) J. Mol. Biol. 222, 581-597): 1.75x10⁸ individual clones for the heavy chain, 9.4x10⁷ clones for V_{κ}, and 5.2x10⁷ clones for V_{λ}. In the second step, the heavy chain fragments were digested from plasmid DNA isolated from the primary V_{H} repertoire, and cloned into the vector containing the light chain repertoires (again separately for PBL and spleen derived repertoire). The libraries were combined using this efficient cloning procedure, to create a naVve Fab repertoire with 3.7x10¹⁰ individual clones (4.3x10¹⁰ recombinant clones, 86% of which have a full-length Fab insert), with 70% of clones harbouring a kappa light chain, 30% a lambda chain. All of 20 clones with full length Fab insert tested scored positive in dot-blot analysis with the 9E10 antibody to indicating an expression level of soluble Fab of at least 0.2 mg/L.

We evaluated the library by selection with different antigens. First, the results from three model antigens, the protein tetanus toxoid, the hapten 2-phenyloxazol-5-one (phOx) (Griffiths, et al., (1984) Nature 312, 271-275, and the peptide MUC1, are discussed. Three rounds of biopanning on tetanus toxoid yielded a diverse set of ELISA positive Fab's, in a series of 47 tetanus toxoid binding Fab's, at least 21 were different with regard to *Bst*NI-fingerprint. Similarly, an extensive panel of phOx-specific Fab's was retrieved after three rounds of panning: at least 24 different clones were identified in a series of 50 ELISA positive clones. Solution capture with biotinylated MUC1 peptide resulted in the selection of 14 different antibody fragments out of 37 ELISA-positive clones selected after 3 rounds.

As a more stringent test panel of antigens to assay the performance of the library, we chose to derive antibodies to three structurally related glycoproteins: human Chorionic Gonadotropin (hCG), human Luteinizing Hormone (hLH) and human Follicle Stimulating Hormone (hFSH) (reviewed in (Cole, (1997) Clin. Chem. 43, 2233-2243)). These hormones are heterodimers sharing an identical α-chain with 92 amino acid residues, but have β-subunits of different composition and length. The β-chain of hCG contains 145 amino acid residues, and the one from hLH only 121 residues, the latter showing 85% homology to β-hCG. The β-chain of hFSH is only 111 amino acids and shares 36% of the residues with hCG. Antibodies that specifically detect hCG have been used extensively in pregnancy tests (Cole, (1997) Clin. Chem. 43, 2233-2243) and for cancer diagnosis (Masure, et al., (1981) J. Clin. Endocrinol. Metab. 53, 1014-1020; Papapetrou, et al., (1980) Cancer 45, 2583-2592). A large set of antibodies to these targets would extend the limited number of hormone specific antibodies (especially against hLH), obtained using the hybridoma technology (Cole, (1997) Clin. Chem. 43, 2233-2243). The human origin of the antibodies might be beneficial when using these for imaging or therapy of testicular and bladder cancer (Masure, et al., (1981) J. Clin.Endocrinol. Metab. 53, 1014-1020; Papapetrou, et al., (1980) Cancer 45, 2583-2592).

Selections were thus performed on biotinylated urinary hCG, recombinant hLH, hFSH and hFSH-CTP (the latter is a chimeric molecule containing the carboxy terminal peptide of β-hCG fused to the β-chain of FSH (Fares, et al., (1992) Proc. Natl. Acad. Sci. U. S. A. 89, 4304-4308)). The highest degree of enrichment in respect to the increase in the number of eluted phage particles in round 3 versus round 1 was found for hCG (10,000-fold), followed by hFSH-CTP (1,000-fold), hFSH (300-fold) and hLH (150-fold). Polyclonal phage of selected populations were tested for binding using sensorchips containing immobilised hormones (Schier, et al., (1996) Hum. Antibodies Hybridomas 7, 97-105). Polyclonal phage selected with hCG showed binding after rounds two and three of selection to all three proteins, i.e., hCG, hLH, and hFSH, with the strongest signal visible for hCG. Similar analysis of the polyclonal phage populations selected for three rounds on hFSH showed a dominance of hFSH-specific binding, while selections on hFSH-CTP yielded binders to both hFSH and hCG. Selections on hLH yielded antibodies reactive with hFSH and hCG. Thus, this polyclonal phage screening provides a rapid test to check the overall quality of the clones in the selected repertoire, and may also be used to guide the choice of the conditions for the next selection round (Schier, et al., (1996) Hum. Antibodies Hybridomas 7, 97-105).

ELISA of monoclonal phage antibodies revealed that three rounds of selection with hCG indeed resulted in the isolation of a high percentage (74%) of clones positive for the gonadotropin. 27% of these clones were hLH cross-reactive; none were reactive against streptavidin. *Bst*NI-fingerprint analysis of the ELISA-positive clones revealed a high degree of diversity (8 different patterns). From a representative hCG-specific (coded CG#4F) and hLH cross-reactive (CG#5C) clone, the specificity was tested in BIAcore using unpurified soluble Fab fragments. Clone CG#4F gave a high response on hCG, with no visible binding to either hLH or hFSH-CTP. In contrast, clone CG#5C bound to hCG and hLH, but not to hFSH-CTP. Western blots, with the different hormones in non-reduced form, showed the specific recognition of the β-subunit of hCG by clone CG#4F, while the cross-reactive clone CG#5C reacted with the β-subunit of both hCG and hLH.

Selection with the hormone hLH resulted in the isolation of hLH-specific and hCG cross-reactive clones. Examination of individual clones from selection round three in ELISA revealed a large fraction of hLH specific clones (69%), and a minor group of cross-reactive clones (16%); no streptavidin reactive clones were selected. Within the group of specific clones, a large array of different species (>21) could be discriminated with fingerprint analysis; however, all cross-reactive species had a single pattern. The unique hLH specificity was confirmed for representative clones LH#2H and LH#3G, shown in surface plasmon resonance; and on western blot. LH#3G only recognises the intact α/β-heterodimer of hLH. Two representative clones of a pan-reactive antibody in ELISA, coded LH#1C and LH#3F, reacted in BIAcore with hFSH-CTP, hCG and hLH, and in western blot analysis with the α-chains from all three hormones.

When hFSH was used as antigen during selection, 6 different antibodies were isolated from the library, with one type, represented by clone FS#8B, dominating the selected population. This Fab only recognised hFSH in BIAcore, and, as western blot analysis demonstrated, in particular its β-unit. Further, the specificity of an α-chain binding clone, SC#2B, was confirmed in BIAcore and western blot.

Upon selection with FSH-CTP 7 different α-chain specific Fab's were identified by fingerprint analysis, from which the clones coded SC#2B, SC#2F, SC#2G and SC#4G were examined in more detail. Immunoblot analysis with the recombinant Fab as detecting antibody confirmed the α-chain specificity.

The affinities and off-rates of affinity purified hCG reactive Fab's LH#1C, SC#2B, LH#3F and CG#5C were determined. The off-rates for most Fab's were in the order of 10⁻² and 10⁻³ s⁻¹. The off-rate values obtained using crude periplasmic fractions were in good agreement with the values found for the purified Fab's, validating the utility of the off-rate screen with unpurified Fab fragments. The affinities, 23 nM and 38 nM for the α-subunit specific antibody LH#1 C and the β-subunit hCG/hLH-cross reactive antibody CG#5C respectively, are comparable to the affinity of antibodies selected from a murine immune phage antibody library (H.d.H., B. Kazemier, *et al.,* unpublished); the top affinity, 2.7 nM for the α-chain specific Fab SC#2B, approaches the values of the best anti-hCG monoclonal antibodies (H.d.H., B. Kazemier, *et al.,* unpublished).

The aim of this procedure is to select and enrich for phage-antibodies to an antigen coated on the surface of immunotubes. The antigen is coated to the immunotube (e.g., a Nunc-immunotube) and incubated with the phage library. Non-bound phage are washed away and the binding phage are eluted, therefore the phage library becomes enriched for phage antibodies that specifically bind the antigen.

The aim of this procedure is to biotinylate proteins or peptides. At neutral pH or above, primary amine-groups react with NHS-SS-Biotin, and N-hydroxysulfosuccimide is released. The N-terminal free NH₂-groups as well as lysines (K) of the protein react with NHS-S-S-Biotin, in this pH range.

NHS-SS-Biotin is a unique biotin analog with an extended spacer arm of approximately 24.3 A in length, the spacer arm of NHS-LC-Biotin is 22.4 A. These long chain analogs reduce steric hindrances associated with binding of biotinylated molecules to avidin or streptavidin.

The presence of the S-S linker in NHS-S-S-Biotin enables disruption of binding using reducing agents (DTT, DTE, β-mercaptoethanol). NHS-LC-Biotin is used when biotinylated protein/peptide is needed that is not sensitive to reducing agents.

The aim of this procedure is to select phage antibodies against a biotinylated antigen. The selection is done in solution, and can be used to select phage antibodies against antigens that are prone to denaturation when coated onto solid surfaces.

First the biotinylated antigen is incubated with the phage antibody library. After addition of the Dynabeads (Dynal) coated with streptavidin, the biotin of the antigen-antibody-complex will bind to the streptavidin. This Dynabead-antigen-antibody-complex is pulled out with a magnet (e.g., a Dynal magnet) and therefore should contain the specific antibodies.

The aim of this procedure is to select for those antibodies out of a library that bind to antigens present in the cell membrane, using adherent growing cells or cells in suspension. The method can be used for selection of antibodies against targets expressed on (tumor) cell lines.

By incubating whole cells, organelles, or membrane fractions with a high variety phage antibody repertoire, such as the Fab Libraries obtained in accordance with the invention (concentrated by PEG precipitation), only (or preferentially) relevant antibodies, to one of the molecules exposed on the surface of the cellular membrane(s), will be retained while not binding phage antibodies are separated from the antibodies bound to the cells, organelles or membrane fractions (by methods well known in the art for separating cells, organelles or membrane fractions from molecules in solution). The retained phage population is enriched for those clones which are specific for cell related molecules. In principle the following factors will positively influence the enrichment of individual clones: Affinity, antigen abundance, and low toxicity of the antibody construct to TG1 host.

The aim of this procedure is to prepare soluble antibody fragments from the periplasm of *E. coli.* In the periplasm there is: less protease activity, less contaminating proteins than in the cytoplasm or supernatants, and the antibody is more concentrated. Therefore, periplasmic preparations are more stable and more pure than culture supernatants.

As a consequence of induction of phagemid containing bacterial cultures in low glucose medium with IPTG, soluble antibody fragments are produced and directed to the periplasm where they are concentrated within 4 hours. Overnight culturing in these circumstances will make the bacterial membrane leaky and antibodies will be found in the supernatant. For preparation of periplasmic fractions, the bacterial cell wall is first lysed by cold osmotic shock (icecold TES) and then rapidly diluted in a chilled solution of low osmotic strength (TES/H₂O) The EDTA makes the outer membrane more permeable, and the cold inhibits protease activity. Subsequently, the bacterial cells are spun down and the supernatant then contains the periplasmic proteins. The antibodies in the periplasmic fraction can be used as a "crude extract" or the antibodies can be purified by conventional means well known in the art, for e.g., those recited in Section 5.6 and 5.7.

The aim of this procedure is to purify antibodies labeled with a His6 tag from periplasmic fractions of Fabs made as described in Example 6.18.

Immobilized metal affinity chromatography (IMAC) for the purification of recombinant 6xHis-tagged proteins under native conditions: Recombinant histidine tagged proteins are captured on a chelated metal containing resin through coordination of free N-atoms of the histidines to the metal (mostly Ni²⁺ or Co ²⁺). After washing away contaminating proteins and other cell constituents, the his-tagged protein is specifically eluted from the resin with imidazol which competes for the binding of histidine-residues to the metal ion.

## Claims

1. Method of making a Fab library , the library comprising a plurality of vectors wherein each vector of the plurality of vectors comprises:
- a first cloning region and a second cloning region, wherein
- each cloning region comprises at least one, for the vector unique, restriction enzyme cleavage site,
- each cloning region being 5' flanked by a ribosome binding site and a signal sequence,
- a polynucleotide encoding an anchor region, located 3' of the second cloning region,
- a polynucleotide encoding a constant heavy chain region or portion thereof located between the vector unique restriction enzyme cleavage site of the second cloning region and the anchor region,
- a polynucleotide encoding a tag,
the method comprising:
introducing into the first cloning region a member of a first plurality of variable polynucleotides, said plurality of variable polynucleotides encoding a first plurality of polypeptides, wherein the member of the first plurality of polynucleotides encodes a polypeptide comprising an antibody light chain variable region possibly followed by an antibody light chain constant region,
separately introducing into the second cloning region of each vector a member of a second plurality of variable polynucleotides, said plurality of variable polynucleotides encoding a second plurality of polypeptides, wherein the member of the second plurality of polynucleotides encodes a polypeptide comprising an antibody heavy chain variable region,
and
introducing each of the vectors into a host cell to provide a plurality of capsid particles each comprising a member of the first plurality of variable polynucleotides and a member of the second plurality of polynucleotides, to thereby make the library of antibodies or fragments thereof.

2. Method according to any of the preceding claims, wherein the plurality of polynucleotides encode a Fab library of at least 10⁹ different Fabs.

3. Method according to claim 1, wherein the plurality of polynucleotides encode a Fab library of at least 10¹⁰ different Fabs.

4. Method according to claim 1, wherein the plurality of polynucleotides encode a Fab library of at least 3.7x 10¹⁰ different Fabs.

5. Method of making according to any of claims 1-4, further comprising the steps of:
amplifying a first plurality of polynucleotides with a first set of primers, and
amplifying a second plurality of polynucleotides with a second set of primers,
wherein each set of primers comprises oligonucleotides designed to be homologous to the 5' and 3' end of polynucleotides encoding antibody variable regions or parts thereof, such that they can be used to amplify variable polynucleotide pools from natural or synthetic sources of genes while retaining all or part of the antibody's antigen combining site.

6. Method according to any of claims 1-5, wherein the anchor region comprises a coat protein.

7. Method according to claim 6, wherein the coat protein is a gene III product.

8. Method according to claim 7, wherein the coat protein comprises minor coat protein III of a filamentous phage f_{d}.

## Patentansprüche

1. Verfahren zum Herstellen einer Fab-Bibliothek, wobei die Bibliothek eine Pluralität von Vektoren umfasst, wobei jeder Vektor der Pluralität von Vektoren umfasst:
- eine erste Klonierungsregion und eine zweite Klonierungsregion, wobei
- jede Klonierungsregion mindestens eine, für den Vektor einmalige Restriktionsenzymspaltstelle umfasst,
- jede Klonierungsregion 5' durch eine Ribosomenbindestelle und eine Signalsequenz flankiert ist,
- ein eine Ankerregion kodierendes Polynukleotid 3' der zweiten Klonierungsregion lokalisiert ist,
- ein eine konstante schwere Kette-Region oder Teil davon kodierendes Polynukleotid zwischen der einmaligen Restriktionsenzymspaltstelle des Vektors der zweiten Klonierungsregion und der Ankerregion lokalisiert ist,
- ein ein Tag kodierendes Polynukleotid,
wobei das Verfahren umfasst:
Einführen eines Mitglieds einer ersten Pluralität von variablen Polynukleotiden in die erste Klonierungsregion, wobei die Pluralität von variablen Polynukleotiden eine erste Pluralität von Polypeptiden kodiert, wobei das Mitglied der ersten Pluralität der Polynukleotide ein Polypeptid kodiert, das eine Antikörper leichte Kette variable Region, möglicherweise gefolgt durch eine Antikörper leichte Kette konstante Region, kodiert,
separates Einführen eines Mitglieds einer zweiten Pluralität von variablen Polynukleotiden in die zweite Klonierungsregion von jedem Vektor, wobei die Pluralität der variablen Polynukleotide eine zweite Pluralität von Polypeptiden kodiert, wobei das Mitglied der zweiten Pluralität der Polynukleotide ein Polypeptid kodiert, das eine Antikörper schwere Kette variable Region umfasst, und
Einführen jedes der Vektoren in eine Wirtszelle, um eine Pluralität von Capsidpartikeln bereitzustellen, die jeweils ein Mitglied der ersten Pluralität von variablen Polynukleotiden und ein Mitglied der zweiten Pluralität von Polynukleotiden umfassen, um dadurch die Bibliothek von Antikörpern oder Fragmenten davon herzustellen.

2. Verfahren nach einem beliebigen der vorhergehenden Ansprüche, wobei die Pluralität der Polynukleotide eine Fab-Bibliothek von mindestens 10⁹ unterschiedlichen Fabs kodiert.

3. Verfahren nach Anspruch 1, wobei die Pluralität der Polynukleotide eine Fab-Bibliothek von mindestens 10¹⁰ unterschiedlichen Fabs kodiert.

4. Verfahren nach Anspruch 1, wobei die Pluralität der Polynukleotide eine Fab-Bibliothek von mindestens 3,7 x 10¹⁰ unterschiedlichen Fabs kodiert.

5. Verfahren zum Herstellen nach einem beliebigen der Ansprüche 1-4, des Weiteren umfassend die Schritte:
Amplifizieren einer ersten Pluralität von Polynukleotiden mit einem ersten Satz an Primern, und
Amplifizieren einer zweiten Pluralität von Polynukleotiden mit einem zweiten Satz von Primern,
wobei jeder Satz von Primern Oligonukleotide umfasst, die entworfen sind, zu dem 5' und 3' Ende der Antikörper variable Regionen oder Teile davon kodierenden Polynukleotiden homolog zu sein, so dass sie verwendet werden können, um variable Polynukleotid-Pools aus natürlichen oder synthetischen Quellen von Genen unter Beibehalten der gesamten oder eines Teils der Antigenbindestelle des Antikörpers zu amplifizieren.

6. Verfahren nach einem beliebigen der Ansprüche 1-5, wobei die Ankerregion ein Hüllprotein umfasst.

7. Verfahren nach Anspruch 6, wobei das Hüllprotein ein Gen-III-Produkt ist.

8. Verfahren nach Anspruch 7, wobei das Hüllprotein das kleinere Hüllprotein III eines filamentösen Phagen f_{d} ist.

## Revendications

1. Procédé de fabrication d'une banque de Fab, la banque comprenant une pluralité de vecteurs dans laquelle chaque vecteur de la pluralité de vecteurs comprend :
- une première région de clonage et une seconde région de clonage, dans lesquelles
- chaque région de clonage comprend au moins un, pour le vecteur unique, site de clivage par enzyme de restriction,
- chaque région de clonage étant flanquée en 5' d'un site de liaison au ribosome et d'une séquence signal,
- un polynucléotide codant pour une région d'ancrage, située en 3' de la seconde région de clonage,
- un polynucléotide codant pour une région constante de chaîne lourde ou une partie de celle-ci située entre le site de clivage par enzyme de restriction du vecteur unique de la seconde région de clonage, et la région d'ancrage,
- un polynucléotide codant pour une étiquette,
le procédé comprenant :
l'introduction dans la première région de clonage d'un élément d'une première pluralité de polynucléotides variables, ladite pluralité de polynucléotides variables codant pour une première pluralité de polypeptides, dans lequel l'élément de la première pluralité de polynucléotides code pour un polypeptide comprenant une région variable de chaîne légère d'anticorps éventuellement suivie d'une région constante de chaîne légère d'anticorps,
l'introduction séparément dans la seconde région de clonage de chaque vecteur, d'un élément d'une seconde pluralité de polynucléotides variables, ladite pluralité de polynucléotides variables codant pour une seconde pluralité de polypeptides, dans lequel l'élément de la seconde pluralité de polynucléotides code pour un polypeptide comprenant une région variable de chaîne lourde d'anticorps,
et
l'introduction de chacun des vecteurs dans une cellule hôte pour fournir une pluralité de particules de capside comprenant chacune un élément de la première pluralité de polynucléotides variables et un élément de la seconde pluralité de polynucléotides, pour fabriquer ainsi la banque d'anticorps ou de fragments de ceux-ci.

2. Procédé selon l'une quelconque des revendications précédentes, dans lequel la pluralité de polynucléotides code pour une banque de Fab d'au moins 10⁹ Fab différents.

3. Procédé selon la revendication 1, dans lequel la pluralité de polynucléotides code pour une banque de Fab d'au moins 10¹⁰ Fab différents.

4. Procédé selon la revendication 1, dans lequel la pluralité de polynucléotides code pour une banque de Fab d'au moins 3,7 x 10¹⁰ Fab différents.

5. Procédé de fabrication selon l'une quelconque des revendications 1 à 4, comprenant en outre les étapes de :
amplification d'une première pluralité de polynucléotides avec un premier jeu d'amorces, et
amplification d'une seconde pluralité de polynucléotides avec un second jeu d'amorces,
dans lequel chaque jeu d'amorces comprend des oligonucléotides conçus pour être homologues aux extrémités 5' et 3' des polynucléotides codant pour des régions variables d'anticorps ou des parties de celles-ci, de telle sorte qu'ils peuvent être utilisés pour amplifier des groupes de polynucléotides variables provenant de sources naturelles ou synthétiques de gènes tout en conservant la totalité ou une partie du site de combinaison à l'antigène de l'anticorps.

6. Procédé selon l'une quelconque des revendications 1 à 5, dans lequel la région d'ancrage comprend une protéine d'enveloppe.

7. Procédé selon la revendication 6, dans lequel la protéine d'enveloppe est un produit de gène III.

8. Procédé selon la revendication 7, dans lequel la protéine d'enveloppe comprend une protéine III d'enveloppe mineure d'un phage filamenteux f_{d}.
